(19) Europäisches Patentamt
European Patent Office
Office européen des brevets

(11) **EP 2 840 175 B1**

(12) **EUROPEAN PATENT SPECIFICATION**

(45) Date of publication and mention
of the grant of the patent:
**26.09.2018 Bulletin 2018/39**

(51) Int Cl.:
*D04H 1/495* (2012.01)       *A61F 13/15* (2006.01)
*A61F 13/49* (2006.01)       *A61F 13/53* (2006.01)
*D04H 1/425* (2012.01)       *A61F 13/511* (2006.01)
*A61F 13/536* (2006.01)      *A61F 13/537* (2006.01)

(21) Application number: **13778480.7**

(22) Date of filing: **27.03.2013**

(86) International application number:
**PCT/JP2013/058980**

(87) International publication number:
**WO 2013/157365 (24.10.2013 Gazette 2013/43)**

(54) **NONWOVEN FABRIC FOR ABSORBENT, AND ABSORBENT ARTICLE**

VLIESSTOFF FÜR ABSORBIERENDEN UND SAUGFÄHIGEN ARTIKEL

TISSU NON TISSÉ POUR PRODUIT ABSORBANT, ET ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.04.2012 JP 2012096970**

(43) Date of publication of application:
**25.02.2015 Bulletin 2015/09**

(73) Proprietor: **Unicharm Corporation
Ehime 799-0111 (JP)**

(72) Inventors:
• **TANGE, Satoru
Kanonji-shi
Kagawa 769-1602 (JP)**

• **OCHI, Kengo
Kanonji-shi
Kagawa 769-1602 (JP)**
• **KONISHI, Takayoshi
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
JP-A- 2006 115 974      JP-A- 2008 025 080
JP-A- 2009 185 408      JP-A- 2009 215 667
JP-A- 2011 208 297      JP-A- 2012 052 253
US-A1- 2007 298 667

**Description**

TECHNICAL FIELD

The present invention relates to a nonwoven fabric for absorbents. More particularly, the present invention relates to a nonwoven fabric for absorbents that is used in absorbent articles such as disposable diapers. The present invention also relates to an absorbent article containing that nonwoven fabric.

BACKGROUND ART

Although absorbents composed of pulp and highly water-absorbent polymers are most commonly used in absorbent articles such as disposable diapers, absorbents are also known that are composed of absorbent paper and highly water-absorbent polymers.

For example, Patent Document 1 discloses a multilayer absorbent paper having a permeable surface layer with which a liquid initially makes contact and one or more diffusing base layers layered thereon, and these layers are each mixed with bulky cellulose fibers to produce paper.

However, the absorbent paper disclosed in Patent Document 1 has a uniform density distribution since it is paper, and although it is bulky, simultaneous to having high density (the permeable layer is described in the examples as having density of 0.11 $g/cm^3$ to 0.14 $g/cm^3$), it is also smooth, and since it further uses heat-meltable adhesive fibers, binder and a papermaking assistant to ensure strength, there are few gaps between fibers resulting in decreased wettability, thereby preventing the transport of large amounts of liquid, while also being unable to demonstrate flexibility.

In addition, Patent Document 2 discloses a liquid diffusing sheet having a liquid-absorbent nonwoven fabric provided with a band-shaped high-density area extending in the lengthwise direction, and this band-shaped high-density area is formed by hot-pressing a liquid-absorbent nonwoven fabric containing heat-fusible fibers.

However, since the absorbent paper described in Patent Document 2 has only a small area where a high-density portion and low-density portion make contact, and the high-density portion is formed by hot-pressing a liquid-absorbent nonwoven fabric containing heat-fusible fibers, simultaneous to the high-density portion having small gaps, hydrophilicity is inferior, and since the action of liquid transfer attributable to variations in density is limited, it is unable to transport large amounts of body fluid.

Prior Art Documents

Patent Documents

Patent Document 1: Japanese Patent No. 3021227
Patent Document 2: Japanese Unexamined Patent Publication No. 2003-235894

DISCLOSURE OF THE INVENTION

Problems to be Solved by the Invention

When a person wears a disposable diaper, although the absorbent in the disposable diaper curves along the body of the wearer and extends from the abdomen to the buttocks after passing through the crotch, when the wearer is standing or sitting down, the crotch is at a low position while the abdomen and buttocks are at a high position. When the wearer has voided while standing or sitting down, the urine is discharged to the crotch and is absorbed and retained in the crotch region of the absorbent, and in the case of an absorbent of the prior art, there was hardly any occurrence of urine flowing counter to gravity and diffusing from the crotch region to the buttocks region of the absorbent. Namely, the only region of the absorbent that was actually used effectively was the crotch region, while the abdomen region and buttocks region were not used effectively.

The present invention provides a nonwoven fabric for absorbents that demonstrates superior diffusing properties by allowing urine to flow counter to gravity and diffuse to the abdomen region and buttocks region of the absorbent even when a wearer voids while standing or sitting down.

Means for Solving the Problems

The inventors of the present invention found that by using a wet spun lace nonwoven fabric and laminating a fiber assembly on the inherent high-density portion of the nonwoven fabric by partially separating from the same nonwoven fabric while simultaneously forming a density gradient in the resulting laminated portion consisting of areas of high density, low density and high density in that order starting from the surface side and continuously forming that density gradient in the machine direction, a nonwoven fabric can be obtained that demonstrates superior diffusing properties, thereby leading to completion of the present invention.

The present invention is a nonwoven fabric for absorbents according to claim 1.

In addition, the present invention is an absorbent article according to claim 7.

More specifically, the present invention is as indicated below.

[1] A nonwoven fabric for absorbents composed of hydrophilic fibers in the form of cellulosic fibers, wherein the non-woven fabric has an upper side and a lower side on the opposite side therefrom,

the non-woven fabric has ridges and grooves in the upper surface extending in the lengthwise direction that alternate in the widthwise direction, when a rectangle that contacts outermost portions of a ridge is drawn in a topographical image of a cross-section in widthwise direction of the nonwoven fabric obtained by X-ray CT, and then the rectangle is divided into three sections in the vertical direction, the three sections being referred to as Y1, Y2, and Y3, respectively, from the bottom, and is divided into five sections in the horizontal direction, the five sections being referred to as X1, X2, X3, X4 and X5, respectively, from the left, and sections X1Y1, X2Y1, X3Y1, X4Y1 and X5Y1 are defined as a site A, sections X1Y2, X1Y3, X2Y3, X4Y3, X5Y3 and X5Y2 are defined as a site B, and sections X2Y2 and X4Y2 are defined as a site C,, and

the proportion of fibers occupying the cross-sectional area of the site B is higher than the proportion of fibers occupying the cross-sectional area of the site C, wherein the proportion of fibers occupying the cross-sectional area of the groove is higher than the proportion of fibers occupying the cross-sectional area of the site B.

[2] The nonwoven fabric described in [1], wherein, when sections X3Y2 and X3Y3 are defined as a site D the proportion of fibers occupying the cross-sectional area of the site D is smaller than the proportion of fibers occupying the cross-sectional area of the site B and larger than the proportion of fibers occupying the cross-sectional area of the site C.

[3] The nonwoven fabric described in any of [1] or [2], wherein tensile strength in the lengthwise direction is 4 N/25 mm or more when dry and 0.4 N/25 mm or more when wet.

[4] The nonwoven fabric described in any of [1] or [3], wherein when the average proportion of fibers occupying the cross-sectional area of all cross-sections in the widthwise direction of the nonwoven fabric is defined as $r_0$, then the proportion of fibers occupying the cross-sectional area of the site A ($r_A$) is $1.2r_0$ or more, the proportion of fibers occupying the cross-sectional area of the site B ($r_B$) is $0.8r_0$ to less than $1.2r_0$, and the proportion of fibers occupying the cross-sectional area of the site C ($r_C$) is less than $0.8r_0$

[5] The nonwoven fabric described in any of [2] or [3], wherein when the average proportion of fibers occupying the cross-sectional area of all cross-sections in the widthwise direction of the nonwoven fabric is defined as $r_0$, then the proportion of fibers occupying the cross-sectional area of the site A ($r_A$) is $1.2r_0$ or more, the proportion of fibers occupying the cross-sectional area of the site B ($r_B$) is $0.8r_0$ to less than $1.2r_0$, the proportion of fibers occupying the cross-sectional area of site D ($r_D$) is $0.5r_0$ to less than $0.8r_0$, and the proportion of fibers occupying the cross-sectional area of the site C ($r_C$) is less than $0.5r_0$.

[6] The nonwoven fabric described in any of [1] to [5], wherein the nonwoven fabric contains beaten pulp and regenerated cellulose at ratios of 20 parts by weight to 40 parts by weight and 10 parts by weight to 70 parts by weight, respectively, and the beaten pulp is fibrillated and has a weighted average fiber length over a range of 1.0 mm to 5.0 mm.

[7] An absorbent article containing a liquid permeable top sheet, a liquid impermeable back sheet and an absorbent interposed between the top sheet and the back sheet, wherein the absorbent contains the nonwoven fabric described in any of [1] to [6].

Effects of the Invention

The nonwoven fabric of the present invention demonstrates superior diffusing properties.

BRIEF DESCRIPTION OF THE DRAWINGS

FIG. 1 is a schematic perspective view of a nonwoven fabric of a first embodiment of the present invention.

FIG. 2 is a schematic transverse cross-sectional view of a nonwoven fabric of a first embodiment of the present invention.

FIG. 3 is a schematic transverse cross-sectional view of a nonwoven fabric of a second embodiment of the present invention.

FIG. 4 is a schematic transverse cross-sectional view of a nonwoven fabric of a third embodiment of the present invention.

FIG. 5 is a drawing for explaining a method for measuring the proportion of fibers occupying a cross-sectional area.

FIG. 6 is a drawing showing an example of a nonwoven fabric production apparatus for producing the nonwoven fabric of the present invention.

FIG. 7 shows an example of a steam nozzle.

FIG. 8 is a drawing for explaining the principle by which sites A to D are formed by steam jet treatment.

FIG. 9 is a schematic transverse cross-sectional view of an example of an absorbent article.

FIG. 10 is a photograph of a cross-section in the widthwise direction of a nonwoven fabric of Comparative Example 1.

FIG. 11 is a photograph of a cross-section in the widthwise direction of a nonwoven fabric of Comparative Example 2.

FIG. 12 is a photograph of a cross-section in the widthwise direction of a nonwoven fabric of Example 1.

FIG. 13 is an XY topographical image of a nonwoven fabric of Example 1.

FIG. 14 is a photograph of a cross-section in the widthwise direction of a nonwoven fabric of Example 2.

FIG. 15 is an XY topographical image of a nonwoven fabric of Example 2.

FIG. 16 is an XY topographical image of a nonwoven fabric of Example 3.

FIG. 17 is a photograph of a cross-section in the widthwise direction of a nonwoven fabric of Example 4.

FIG. 18 is an XY topographical image of a nonwoven fabric of Example 4.

BEST MODE FOR CARRYING OUT THE INVENTION

The present invention is a nonwoven fabric for absorbents composed of hydrophilic fibers in the form of cellulosic fibers, wherein the nonwoven fabric has an upper surface and a lower surface on the opposite side therefrom, the nonwoven fabric has ridges and grooves in the upper surface extending in the lengthwise direction that alternate in the widthwise direction, a site A extends over the lower surface side of the ridges, a site B extends over the sidewalls and top of the upper surface side of the ridges, a site C is present interposed between the site A and the site B, the proportion of fibers occupying the cross-sectional area of the site A is higher than the proportion of fibers occupying the cross-sectional area of the site B, and the proportion of fibers occupying the cross-sectional area of the site B is higher than the proportion of fibers occupying the cross-sectional area of the site C.

Although the following provides an explanation of the present invention with reference to the drawings, the present invention is not limited to that described in the drawings.

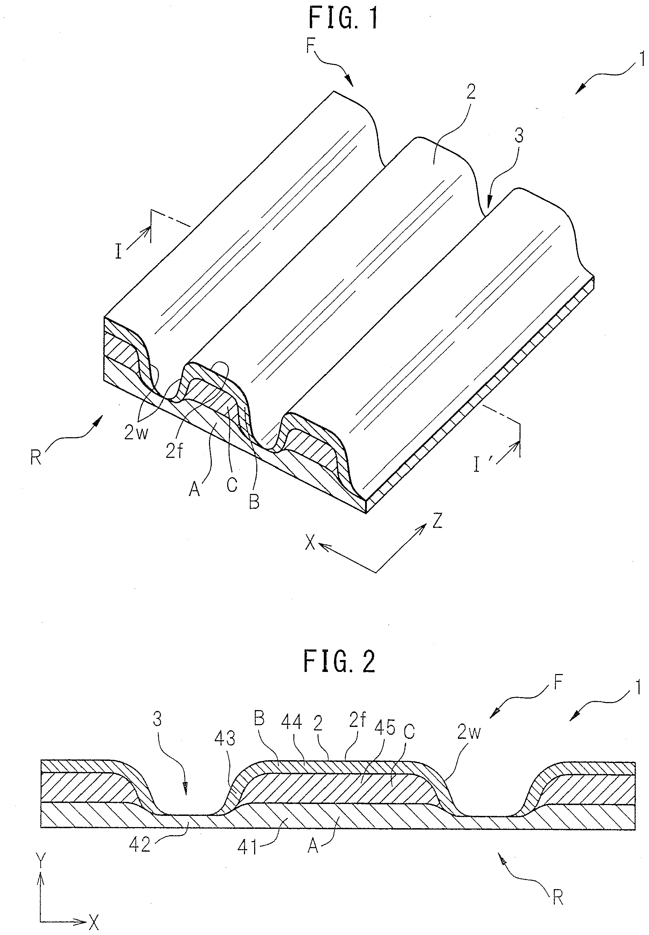

FIG. 1 is a schematic perspective view of a nonwoven fabric of a first embodiment of the present invention. FIG. 2 is a cross-sectional view (schematic diagram) in the widthwise direction taken along line I-I' of the nonwoven fabric of FIG. 1. A nonwoven fabric 1 has an upper surface F and a lower surface R on the opposite side therefrom. The nonwoven fabric 1 has ridges 2 and grooves 3 on the upper surface F extending in the lengthwise direction (Z direction) that alternate in the widthwise direction (X direction). A site A extends over the lower surface R side of the ridges 2, a site B extends over sidewalls 2w and top 2f of the upper surface F side of the ridges 2, and a site C is present interposed between the site A and the site B. The proportion of fibers occupying the cross-sectional area of site A ($r_A$) is higher than the proportion of fibers occupying the cross-sectional area of site B ($r_B$), and the proportion of fibers occupying the cross-sectional area of site B ($r_B$) is higher than the proportion of fibers occupying the cross-sectional area of site C ($r_C$). The grooves 3 are composed at the site A extending from the ridges 2.

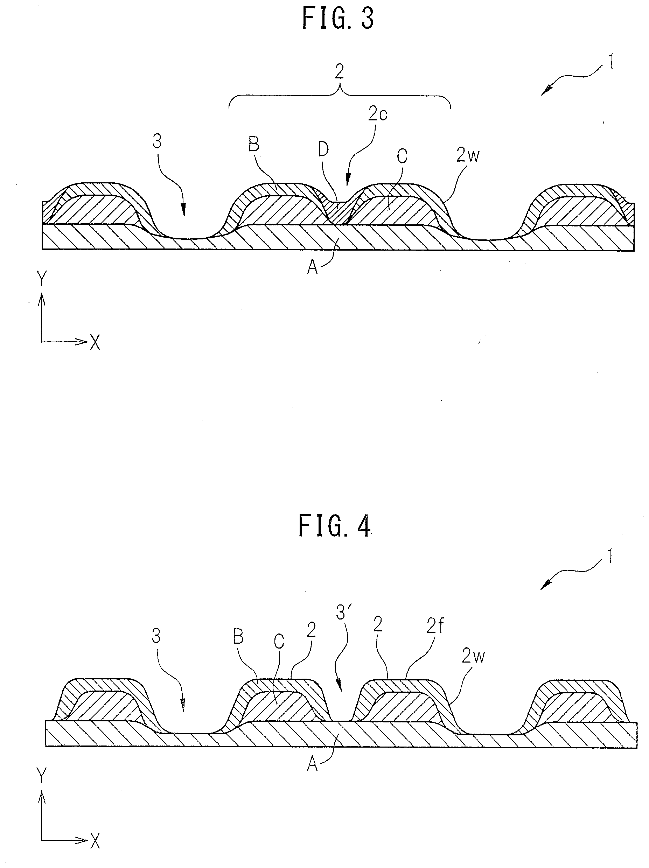

FIG. 3 is a cross-sectional view (schematic diagram) in the widthwise direction of a nonwoven fabric of a second embodiment of the present invention. In this embodiment, a site D is further present at a central portion 2c of the ridges in the widthwise direction, and the proportion of fibers occupying the cross-sectional area of the site D ($r_D$) is smaller than the proportion of fibers occupying the cross-sectional area of the site B ($r_B$) but larger than the proportion of fibers occupying the cross-sectional area of the site C ($r_C$).

FIG. 4 is a cross-sectional view (schematic diagram) in the widthwise direction of a nonwoven fabric of a third embodiment of the present invention. This embodiment is the same as the first embodiment with the exception of having two types of grooves 3 and 3'. The grooves 3' are narrower and shallower than the grooves 3.

In other words, in a cross-section of the nonwoven fabric of the present in the widthwise direction, the proportion of fibers occupying the cross-sectional area of a sidewall 43 of a ridge is smaller than the proportion of fibers occupying the cross-sectional area of a bottom 41 of a ridge and the proportion of fibers occupying the cross-sectional area of a groove 42, and the proportion of fibers occupying the cross-sectional area of an interior 45 of a ridge is smaller than the proportion of fibers occupying the cross-sectional area of a surface layer 44 of a ridge. Preferably, the proportion of fibers occupying the cross-sectional area of the surface layer 44 of a ridge is roughly equal to the proportion of fibers occupying the cross-sectional area of the sidewall 43 of a ridge or smaller than the proportion of fibers occupying the cross-sectional area of the sidewall 43 of a ridge.

In the present invention, the proportion of fibers occupying a cross-sectional area refers to the ratio of the area occupied by fibers in a measured region determined in a topographical image of the nonwoven fabric obtained by X-ray CT to the area of that measured region.

The proportion of fibers occupying a cross-sectional area was measured in the manner indicated below.

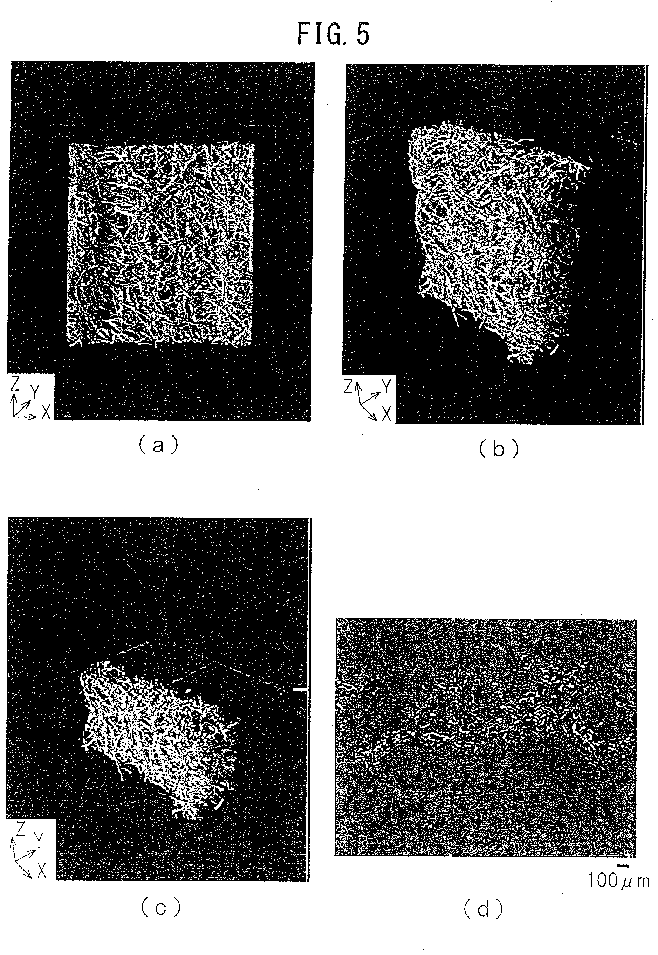

(1) X-ray CT Observation of Nonwoven Fabric Cross-Section
and Image Analysis
Non-destructive observation of the internal structure of a sample is carried out using X-ray CT. Topographical images are obtained by analyzing the resulting data.
* The sample is rotated 360 degrees. At that time, data is acquired in the manner of acquiring X-ray absorption data during the first rotation followed by acquiring the same data during the second rotation of the sample and so forth.
(2) Three-Dimensional Observation by Analytical Software
(Acquisition of Three-Dimensional Images and Topographical Images)
Three-dimensional images (FIGS. 5(a) and 5(b)) were generated from the topographical images obtained by X-ray CT to confirm the internal status (XY topographical image) (FIG. 5(d)) in an arbitrary XY cross-section (FIG. 5(c)).
* A topographical image refers to a two-dimensional image. A three-dimensional image is generated by superimposing topographical images.
(3) Measurement of Fiber Area by Analytical Software
A measured region was extracted from an XY topographical image obtained by X-ray CT followed by measurement of fiber area.
The outermost fibers present in a portion in which fibers composing the XY topographical image used for the measured region as determined from three-dimensional images and XY topographical images mainly form ridges were connected and used for the range over which ridges are formed.
A rectangle was then drawn that contacts the outermost portions of the ridges followed by dividing into three sections in the vertical direction and dividing into five sections in the horizontal direction, measuring the cross-sectional areas of those regions, the cross-sectional area of ridges present in a quadrilateral and the cross-sectional area of fibers, and calculating the proportion of fibers occupying a cross-sectional area for each site.
Cross-sections were extracted at locations that were free of irregular portions whenever possible, and the average was determined after making measurements at 10 locations.
(4) Details of Determination of Fiber Cross-Sectional Area
Two-dimensional evaluations can also be carried out with three-dimensional volume rendering software.
Area can be calculated by extracting topographical images.

[Border Determination]

A target region is designated (such as one of the 15 sections).
The contrast of a fiber portion is extracted.
The contrast of a non-fiber portion is extracted.
White, black and gray portions are binarized (white and black) and interfaces are generated by automatically analyzing with software.

[Calculation of Area]

After using software to analyze those pixels corresponding to ridges and those pixels corresponding to fiber portions present within the rectangle surrounding the ridges while making contact therewith, pixels were converted to units of $\mu m^2$ followed by calculating area from each of the pixel values (automatically calculated with software).

[Calculation of Proportion of Cross-Sectional Area]

The proportion of cross-sectional area was determined by being separately calculated by the person performing measurements from the area of the region as calculated with software and the fiber cross-sectional area. The calculation formula is as indicated below.

```
(Proportion of fibers occupying cross-sectional area
r) (%)    = (fiber cross-sectional area of target region)
      /
      (cross-sectional area of ridges present within
      quadrilateral of target region) × 100
```

(5) Measurement Conditions

X-ray tube voltage: 40 kV
X-ray tube current: 15 μA
Pixel number: 512 × 512 pixels
Viewing field size: 2.5 mmφ × 2.5 mm h

(6) Equipment Used

Three-dimensional measurement X-ray CT System: TDM1000-IS/SP (Yamato Scientific Co., Ltd.)

Three-dimensional volume rendering software: VG-Studio MAX (Nihon Visual Science, Inc.)

In the nonwoven fabric of the present invention, the proportion of fibers occupying a cross-sectional area in a cross-section in the widthwise direction is such that site A has the highest proportion, site B has the next highest proportion, site D has the next highest proportion and site C has the lowest proportion. The proportions of fibers occupying the cross-sectional areas of site A, site B, site D and site C are only required to satisfy this size relationship, and there are no particular limitations on the absolute values of the proportions of fibers occupying these cross-sectional areas.

However, if the average proportion of fibers occupying the cross-sectional areas of all cross-sections of the nonwoven fabric in the widthwise direction is defined as $r_0$, then preferably the proportion of fibers occupying the cross-sectional area of site A ($r_A$) is $1.2r_0$ or more, the proportion of fibers occupying the cross-sectional area of site B ($r_B$) is $0.8r_0$ to less than $1.2r_0$, and the proportion of fibers occupying the cross-sectional area of site C ($r_C$) is less than $0.8r_0$. More preferably, the proportion of fibers occupying the cross-sectional area of site A ($r_A$) is $1.2r_0$ to less than $2.0r_0$, the proportion of fibers occupying the cross-sectional area of site B ($r_B$) is $0.8r_0$ to less than $1.2r_0$, and the proportion of fibers occupying the cross-sectional area of site C ($r_C$) is $0.1r_0$ to less than $0.8r_0$.

In addition, when the site D is present, preferably the proportion of fibers occupying the cross-sectional area of site A ($r_A$) is $1.2r_0$ or more, the proportion of fibers occupying the cross-sectional area of site B ($r_B$) is $0.8r_0$ to less than $1.2r_0$, the proportion of fibers occupying the cross-sectional area of site D ($r_D$) is $0.5r_0$ to less than $0.8r_0$ and the proportion of fibers occupying the cross-sectional area of site C ($r_C$) is less than $0.5r_0$. More preferably, the proportion of fibers occupying the cross-sectional area of site A ($r_A$) is $1.2r_0$ to less than $2.0r_0$, the proportion of fibers occupying the cross-sectional area of site B ($r_B$) is $0.8r_0$ to less than $1.2r_0$, the proportion of fibers occupying the cross-sectional area of site D ($r_D$) is $0.5r_0$ to less than $0.8r_0$ and the proportion of fibers occupying the cross-sectional area of site C ($r_C$) is $0.1r_0$ to less than $0.5r_0$.

For example, in the case the average proportion of fibers occupying the cross-sectional areas of all cross-sections of a nonwoven fabric in the widthwise direction $r_0$ is 9%, preferably the proportion of fibers occupying the cross-sectional area of site A ($r_A$) is 10.8% to less than 18%, the proportion of fibers occupying the cross-sectional area of site B ($r_B$) is 7.2% to less than 10.8% and the proportion of fibers occupying the cross-sectional area of site C ($r_C$) is 0.9% to less than 7.2%. In addition, when the site D is present, the proportion of fibers occupying the cross-sectional area of site A ($r_A$) is 10.8% to less than 18%, the proportion of fibers occupying the cross-sectional area of site B ($r_B$) is 7.2% to less than 10.8%, the proportion of fibers occupying the cross-sectional area of site D ($r_D$) is 4.5% to less than 7.2% and the proportion of fibers occupying the cross-sectional area of site C ($r_C$) is 0.9% to less than 4.5%.

Whether or not a nonwoven fabric satisfies the requirements of the proportions of fibers occupying cross-sectional areas stipulated in the present invention can be determined by connecting the outermost fibers present in a portion in which fibers composing a topographical image used for the measured region, as determined from topographical images obtained by X-ray CT, mainly form ridges and using for the range over which ridges are formed, drawing a rectangle that contacts the outermost portions of the ridges followed by dividing into three sections in the vertical direction and dividing into five sections in the horizontal direction, measuring the cross-sectional areas of each of the 15 sections, the cross-sectional area of ridges present in each sections and the cross-sectional area of fibers, and calculating the proportion of fibers occupying the cross-sectional area for each section.

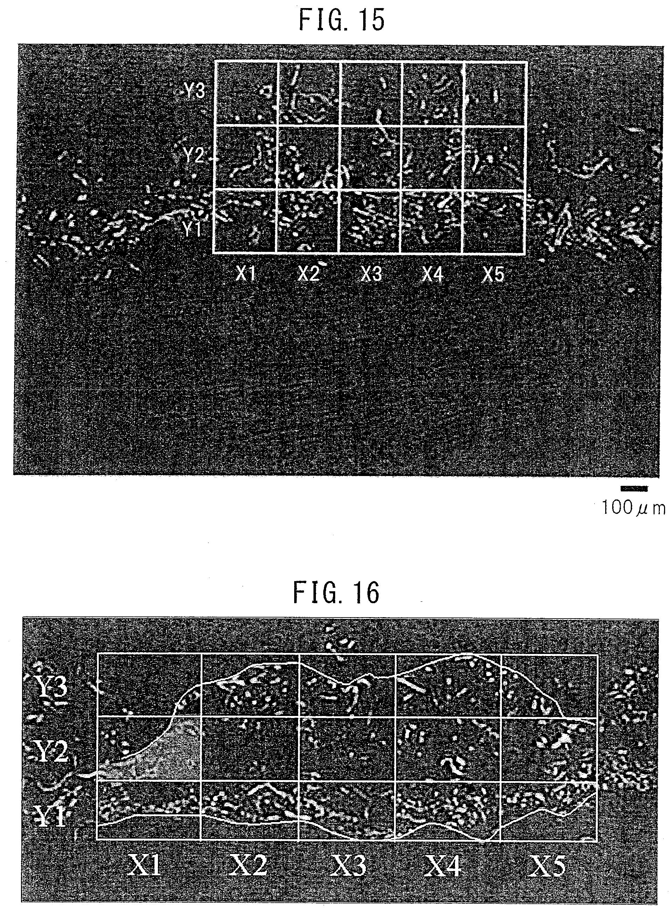

Although FIG. 16 is an example of a topographical image of a cross-section in the widthwise direction of the nonwoven fabric of the present invention, in this topographical image, in the case section X1Y1, section X2Y1, section X3Y1, section X4Y1 and section X5Y1 constitute site A, section X1Y2, section X1Y3, section X2Y3, section X4Y3, section X5Y3 and section X5Y2 constitute site B, and section X2Y2 and section X4Y2 constitute site C, the requirements of site A where the proportion of fibers occupying the cross-sectional area is the highest, site C where the proportion of fibers occupying the cross-sectional area is the lowest, and site B having a proportion of fibers occupying the cross-sectional area that is intermediate to the proportion of fibers occupying the cross-sectional area of site A and the proportion of fibers occupying the cross-sectional area of site C being present in a cross-section in the widthwise direction, and the surface of the ridges being composed at site A, the sidewalls of the ridges being composed at site B, one side of site C contacting site B, and the opposite side on the other side of site C contacting site A, can be considered to be satisfied.

Moreover, in the case section X3Y3 constitutes site B, then the requirement of the surface of the ridges being composed at site B can be considered to be satisfied.

In addition, in the case section X1Y1, section X2Y1, section X3Y1, section X4Y1 and section X5Y1 constitute site A, section X1Y2, section X1Y3, section X2Y3, section X4Y3, section X5Y3 and section X5Y2 constitute site B, section X2Y2 and section X4Y2 constitute site C, and section X3Y2 and section X3Y3 constitute site D, the requirements of site A where the proportion of fibers occupying the cross-sectional area is the highest, site C where the proportion of fibers occupying the cross-sectional area is the lowest, site B having a proportion of fibers occupying the cross-sectional area that is intermediate to the proportion of fibers occupying the cross-sectional area of site A and the proportion of fibers occupying the cross-sectional area of site C and site D having a proportion of fibers occupying the cross-sectional area that is intermediate to the proportion of fibers occupying the cross-sectional area of site B and the proportion of fibers occupying the cross-sectional area of site C being present in a cross-section in the widthwise direction, and the surface of the ridges being composed at site A, the sidewalls of the ridges being composed at site B, one side of site C contacting site B and the opposite side on the other side of site C contacting site A, the central portion in the widthwise direction of the ridges being composed at site D, and site C being surrounded by site A, site B and site D, can be considered to be satisfied.

Since the nonwoven fabric of the present invention has site A, the suctioning height of the base paper is guaranteed. Moreover, the nonwoven fabric has interfaces formed due to variable density. Those interfaces are regions where two differences in density are continuous (change in density and connecting holes). As a result, a capillary phenomenon is presumed to be promoted at the interfaces, and migration of liquid is thought to occur from site C (sparse) to site A and site B (dense). This means that, since migration of liquid can be presumed to occur more easily the larger the number of interfaces, liquid migration, namely capillary rise height, is thought to be greater in the present invention having a large number of interfaces of varying densities than in the case of a simple difference in density as in the comparative examples to be subsequently described. In terms of a phenomenon, it appears as if site C acts as a new liquid surface.

In the nonwoven fabric of the present invention, the liquid absorption height of artificial urine after 5 minutes as determined in a water absorbency test according to the Klemm method is preferably 130 mm or more, more preferably 135 mm or more and even more preferably 140 mm or more, while the water absorption multiple is preferably 2.4 times or more, more preferably 2.6 times or more and even more preferably 3.0 times or more. If the liquid absorption height is excessively low, the absorbent is unable to be moistened efficiently. If the water absorption multiple is excessively low, liquid cannot be efficiently allowed to migrate to the pulp/highly water absorbent polymer that forms an absorbent core. A water absorbency test according to the Klemm method can be carried out in accordance with JIS P 8141:2004. The detailed procedure thereof will be subsequently described.

Since paper is normally produced by going through steps such as sheet formation (by containing heat-fusible fibers and binder), drying and thermocompression bonding as desired, it has a uniform cross-sectional structure, typically has low density, has large gaps between fibers, has a large capillary size, has low suctioning height and the suctioned amount is thought to increase. However, wet spunlace nonwoven fabric adopts a variable density structure as a result of going through sheet formation, water-jetting and drying steps (entangled portions become dense by hydroentanglement). Due to the subsequent drying step, the entire nonwoven fabric becomes bulky and sparse portions are formed due to the bulky state of portions other than hydroentangled portions. Thus, since a variable density structure has already been formed in the widthwise direction (X direction), when areas of low density form between the sparse entangled portions, simultaneous to the suctioned amount increasing, dense entangled portions are supplied with liquid from between the entangled portions and this is thought to result in a further increase in suctioning height. Moreover, as a result of steam jet treatment, variations in density spread in the direction of thickness (Y direction) resulting in the formation of a dense-sparse-dense state and both suctioned volume and suctioning height increase.

The density of the nonwoven fabric of the present invention is preferably 50 mg/cm$^3$ to 200 mg/cm$^3$, more preferably 50 mg/cm$^3$ to 150 mg/cm$^3$ and even more preferably 60 mg/cm$^3$ to 150 mg/cm$^3$. If density is excessively low, adequate strength cannot be ensured. If density is excessively high, capillaries required for liquid diffusion cannot be adequately formed.

Density can be adjusted by controlling basis weight and thickness according to water jet treatment and drying treatment conditions in a wet spunlace nonwoven fabric. The greater the water pressure and number of columnar flows during water jet treatment, the higher the density, and during drying, a state in which one side is free in the manner of an oven dryer, or a state in which a web is passed between a dryer cylinder and a single-sided blanket in the manner of Yankee dryer followed by crimping using pressing force, can be created, and thickness can be controlled by adjusting the conditions thereof. In addition, density can also be controlled according to processing conditions such as steam jet treatment pressure.

The tensile strength in the lengthwise direction of the nonwoven fabric of the present invention when dry is preferably 4 N or more, more preferably 5 N to 15 N and even more preferably 7 N to 15 N per 25 mm of width. Furthermore, in the present description, tensile strength in the lengthwise direction per 25 mm of width when dry may simply be referred to as "dry strength" and the units thereof may be represented by "N/25 mm".

In addition, the tensile strength in the lengthwise direction of the nonwoven fabric of the present invention when wet is preferably 0.4 N or more, more preferably 0.7 N or more and even more preferably 1.6 N or more per 25 mm of width. Furthermore, in the present description, tensile strength in the lengthwise direction per 25 mm of width when wet may simply be referred to as "wet strength" and the units thereof may be represented by "N/25 mm".

If dry strength is excessively low, the nonwoven fabric may ends up tearing during processing resulting in poor productivity. When considering use within the core wrap of an absorbent, if wet strength is excessively low, the nonwoven fabric may be damaged while an absorbent article is worn, thereby impairing the inherent absorption function of absorption, diffusion and distribution of body liquid in a highly water-absorbent polymer. As a result, there is the risk of this leading to leakage and skin problems.

The tensile strength of a nonwoven fabric can be adjusted by controlling the type of fiber that composes the nonwoven fabric, fiber length, beating degree, conditions of water jet treatment and the like. In a wet nonwoven fabric (obtained by forming the fibers into a sheet in a water bath, entangling by water jetting and drying), in the case of having an entanglable fiber length, fiber entanglement occurs due to water jetting and sheet strength improves. In the case of rayon, dry strength improves due to an increase in the number of nodes attributable to water jetting. In the case of beaten pulp, in addition to having hydrogen bonds inherently possessed by the pulp, the number of microfibrils increases due to beating, thereby resulting in an increase in the degree of hydrogen bonding and greater strength in comparison with unbeaten pulp. In addition, microfibrils entangle with other fibers as a result of water jetting, thereby resulting in improved dry strength.

The following provides an explanation of a method for producing the nonwoven fabric of the present invention.

First, hydrophilic fibers in the form of cellulosic fibers are used as raw material, a fiber web is formed on a conveyor surface by a wet method, and water jet treatment is then carried out on the web to produce a wet spunlace nonwoven fabric. Next, the wet spunlace nonwoven fabric is subjected to steam jet treatment from the side of the conveyor surface during water jet treatment. At this time, the fibers are pushed up at fixed intervals by steam jetting as a result of being subjected to interference between the nozzle pitch and conveyor mesh pattern, resulting in the formation of portion of lower density than the state of the original wet spunlace nonwoven fabric. The amount the fibers are pushed up is large when the steam jet makes the strongest contact, and the ends of the fibers that have been pushed up land farther away than in the original wet spunlace nonwoven fabric. As a result, high density portions of the original wet spunlace nonwoven fabric, medium density portions composed of fibers that have been pushed up, and low density portions located between the high density portions and medium density portions are formed. As a result, one or two ridges are formed, which are brought closer in the direction in which they face due to jetting of steam from two nozzle openings, between grooves (machine direction) of the high density portions remaining after the fibers have been pushed up by steam jetting. The pattern of the ridges can be altered according to the nozzle pattern, conveyor mesh pattern or pressure during steam jetting.

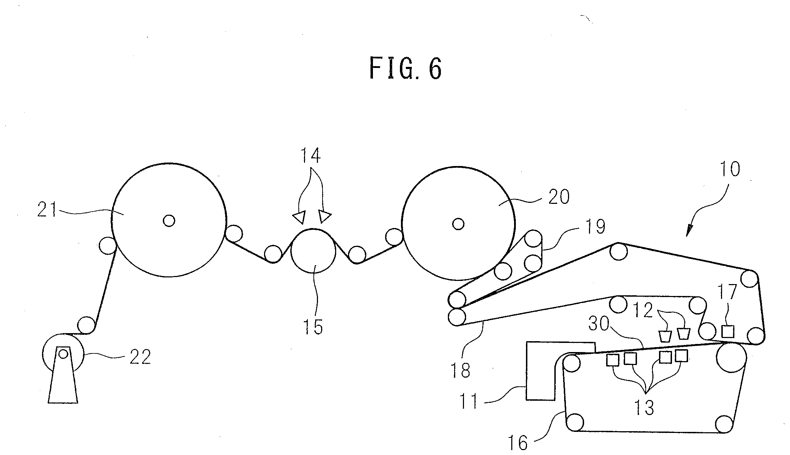

FIG. 6 is a drawing showing an example of a nonwoven fabric production apparatus 10 for producing the nonwoven fabric of the present invention. The following provides a more detailed explanation of the method for producing the nonwoven fabric of the present invention with reference to FIG. 6.

First, a mixture of hydrophilic fibers in the form of cellulosic fibers and water is supplied to and deposited on a web forming conveyor 16 by a raw material supply head 11. A wire mesh and the like can be used for the conveyor 16. Fibers containing water deposited on the conveyor 16 are suitably dehydrated by a suction box 13 to form a web 30. The web 30 passes between two water jet nozzles 12 arranged above the conveyor 16 and two suction boxes 13 arranged at locations opposing the water jet nozzles 12 with the conveyor 16 interposed there between that recover water sprayed from the water jet nozzles 12. At this time, the web 30 is subjected to water jetting from the water jet nozzles 12, fibers of the web 30 are entangled and the strength of the web 30 increases, thereby allowing the obtaining of a wet spunlace nonwoven fabric.

The distance between the ends of the water jet nozzles 12 and the upper surface of the web 30 is preferably 5 mm to 50 mm, more preferably 10 mm to 30 mm and even more preferably 10 mm to 25 mm. If the distance between the ends of the water jet nozzles 12 and the upper surface of the web 30 is too small, problems may occur such as the texture of the web being easily disrupted by the force of water jetting or fibers that have been thrown back by the force of water jetting becoming easily adhered to the nozzles. In addition, if the distance between the ends of the water jet nozzles 12 and the upper surface of the web 30 is too large, treatment efficiency may decrease significantly resulting in the problem of weakening of fiber entanglement.

The aperture of the water jet nozzles 12 is preferably 70 μm to 200 μm, more preferably 90 μm to 150 μm and even more preferably 90 μm to 110 μm. If the aperture of the water jet nozzles 12 is excessively small, problems may occur such as the nozzles being easily clogged. In addition, if the aperture of the water jet nozzles 12 is excessively large, the problem of poor treatment efficiency may occur.

The opening pitch of the water jet nozzles 12 (distance between the centers of adjacent openings) is preferably 0.5 mm to 1.0 mm. If the opening pitch of the water jet nozzles 12 is excessively small, the pressure resistance of the nozzles decreases which may result in the problem of nozzle damage. In addition, if the opening pitch of the water jet nozzles is excessively large, the problem of inadequate fiber entanglement may occur.

Following water jet treatment, the web 30 is transferred to a web transport conveyor 18 by a suction pickup 17, subsequently transferred to a web transport conveyor 19, and then transferred to a drying drum 20. The drying drum 20 is, for example, a Yankee dryer, and the web 30 is dried by adhering to a steam-heated drum. The moisture content of the web 30 can be adjusted prior to entering the steam jet treatment step. The moisture content of the web 30 prior to entering the steam jet treatment step is preferably 10% to 45% and more preferably 20% to 40%. Here, moisture content (%) refers to the number of grams of water contained in 100 g of the total weight of the water-containing web 30. If the moisture content of the web 30 is excessively low, hydrogen bonding strength between fibers of the web 30 becomes excessively strong, and the amount of energy required to break up the fibers of the web 30 by steam jetting to be subsequently described becomes extremely large. Conversely, if the moisture content of the web 30 is excessively high, the amount of energy required to dry the web 30 to a prescribed moisture content or lower following steam jetting to be subsequently described becomes extremely large.

Next, the web 30 is sent to a steam jet treatment step. In the steam jet treatment step, the web 30 moves onto the mesh-like outer peripheral surface of a cylindrical suction drum 15. At this time, steam is sprayed onto the web 30 from steam nozzles 14 arranged above the outer peripheral surface of the suction drum 15. Although two rows of steam nozzles 14 are shown in FIG. 6, one or three or more rows may be used. In the case of the non-woven fabric production apparatus of FIG. 6, the side of the web that is sprayed with steam is on the opposite side (F side) from the side that is sprayed with the water jet. The suction drum 15 contains a built-in suctioning device, and steam sprayed from the steam nozzles 14 is aspirated by that suctioning device. Ridges and grooves are formed in the F side of the web 30 as result of steam jets sprayed from the steam nozzles 14.

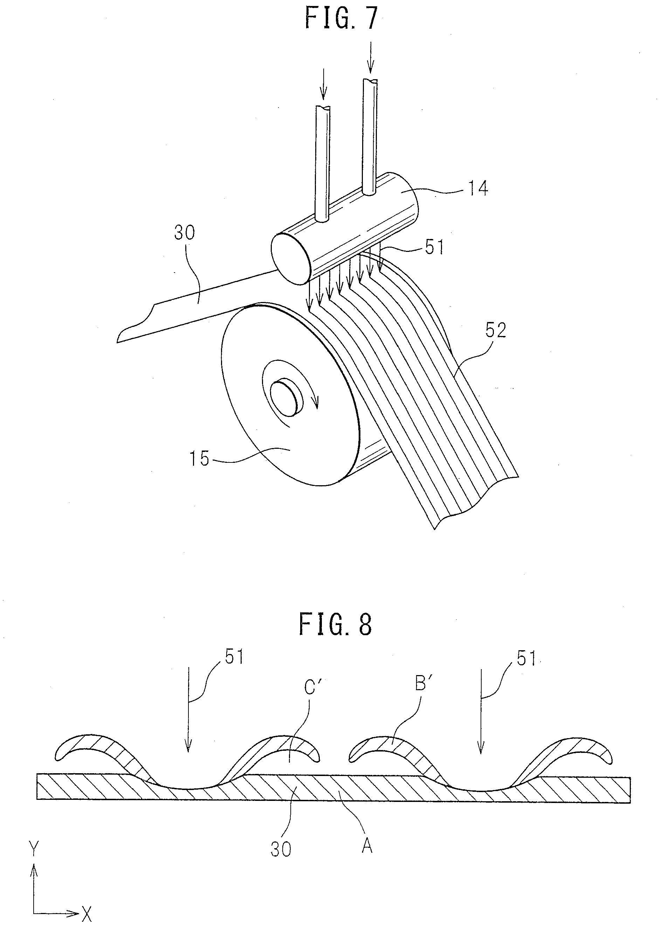

An example of the steam nozzles 14 arranged above the suction drum 15 is shown in FIG. 7. FIG. 7 shows an example of a single row of the steam nozzles 14. The steam nozzles 14 spray a plurality of steam jets 51 arranged in the widthwise direction (X direction) of the web 30 towards the web 30. As a result, a plurality of grooves 52 extending in the machine direction (Z direction) are formed in the widthwise direction (X direction) of the web 30 in the F side of the web 30, and ridges are formed between the grooves.

Although the principle of the formation of sites A to D simultaneous to the formation of ridges 2 and grooves 3 when steam is sprayed onto the web 30 is explained below, this principle does not limit the present invention. FIG. 8 is a drawing for explaining the principle of the formation of sites A to D by steam jet treatment. As shown in FIG. 8, when the steam jets 51 contact the web 30, fibers at those portions where the steam jets 51 make contact are broken up and pushed aside resulting in the formation of grooves, while the fibers that have been pushed aside are thrown to both sides of the grooves resulting in the formation of projections B'.

In the case the pressure of the steam jets 51 is low, the end of the projections B' land on the surface of the web 30 comparatively close to the locations contacted by the steam jets 51, the projections B' become site B, the cavities below the projections B' become site C and the portions where the fibers were not moved by steam jetting form site A, thereby resulting in the formation of a nonwoven fabric as shown in FIG. 4.

If the pressure of the steam jets 51 becomes high, since the fibers that have been pushed aside are thrown farther away, the projections B' become longer, the ends of adjacent projections B' collide with each other at those locations where the ends of the projections B' land, and the portions where they collide become site D, the projections B' become site B, the cavities C' below the projections B' become site C and those portions where the fibers were not moved by steam jetting form site A, thereby resulting in the formation of a nonwoven fabric as shown in FIG. 3.

If the pressure of the steam jets 51 becomes even higher, since the fibers that have been pushed aside are thrown even farther, the projections B' become even longer, the ends of adjacent projections B' become connected prior to landing, and the connected projections B' become site B, the cavities C' below the projections B' become site C, and those portions where the fibers were not moved by steam jetting form site A, thereby resulting in the formation of a nonwoven fabric as shown in FIG. 2.

The pressure of steam sprayed from the steam jet nozzles 14 is preferably 0.3 MPa to 1.5 MPa. If the steam pressure is excessively low, ridges of adequate height may not be formed. In addition, if the steam pressure is excessively high, considerably large holes may be formed in the web 30 or the web 30 may be damaged or blown away.

The suction force by which the web is suctioned by the surface of the suction drum by the suction boxes 13 used to aspirate steam sprayed from the steam nozzles 14 is preferably 1 kPa to 12 kPa. If the suction force of the surface of the suction drum is excessively small, the steam is unable to be completely aspirated causing it to be blown upward resulting in the problem of the occurrence of a dangerous situation. In addition, if the suction force of the surface of the suction drum is excessively large, the problem may occur in which the amount of fiber that falls into the suction drum increases.

The distance between the ends of the steam nozzles 14 and the upper surface of the web 30 is preferably 1.0 mm to 10 mm. If the distance between the ends of the steam nozzles 14 and the upper surface of the web 30 is excessively small, problems may occur such as holes forming in the web 30 or the web 30 being damaged or blown away. In addition, if the distance between the ends of the steam nozzles 14 and the upper surface of the web 30 is excessively large, the force required to form grooves in the surface of the web 30 during steam jetting ends up being dissipated, thereby resulting in poor efficiency when forming grooves in the surface of the web 30.

The aperture of the steam nozzles 14 is preferably 150 $\mu$m to 1000 $\mu$m, more preferably 200 $\mu$m to 800 $\mu$m and even more preferably 250 $\mu$m to 600 $\mu$m. If the aperture of the steam nozzles 14 is excessively small, the amount of energy generated is insufficient which may result in the problem of being unable to adequately push aside the fibers. In addition, if the aperture of the steam nozzles 14 is excessively large, the amount of energy generated becomes excessively large, which may result in the problem of causing excessive damage to the base material.

The opening pitch of the steam nozzles 14 (distance between the centers of adjacent openings) is preferably 1.0 mm to 5.0 mm, more preferably 1.5 mm to 4.5 mm and even more preferably 2.0 mm to 4.0 mm. If the opening pitch of the steam nozzles 14 is excessively small, there is the risk of site C not being formed. Conversely, if the opening pitch of the steam nozzles 14 is excessively large, the number of ridges decreases resulting in the risk of being unable to obtain adequate diffusing properties.

Following steam jet treatment, the web is transferred to a drying drum 21. The drying drum 21 is also, for example, a Yankee dryer and the web 30 is dried by being adhered to a steam-heated drum. The web 30 is preferably sufficiently dry after having passed over the drying drum 21. The dried web 30 is then wound onto a winder 22 in the form of a nonwoven fabric.

The nonwoven fabric of the present invention is composed of hydrophilic fibers in the form of cellulosic fibers. Examples of hydrophilic fibers in the form of cellulosic fibers include pulp and recycled cellulose fibers. Unbeaten pulp or beaten pulp can be used for the pulp.

The nonwoven fabric of the present invention preferably contains beaten pulp and regenerated cellulose at 20 parts by weight to 40 parts by weight and 10 parts by weight to 70 parts by weight, respectively, and the beaten pulp is preferably fibrillated and has a weighted average fiber length within the range of 1.0 mm to 5.0 mm. "Fibrillated" refers to a microfiber portion having a fiber diameter at the submicron level partially separating from the surface of the body portion of the fibers and extending from the surface of the body portion of the fibers.

Beaten pulp can be formed by free beating or wet beating pulp. More specifically, beaten pulp can be formed by, for example, vigorously agitating unbeaten pulp in water by placing in a mixer or passing the unbeaten pulp through a pulper, disc refiner or beater and the like.

Furthermore, the surface of the fibers of the beaten pulp is preferably fibrillated, or in other words, fluffed, without causing a significant change in fiber length both in the case of free beaten pulp and wet beaten pulp. This is because, if fiber length becomes short, the nonwoven fabric tends to become paper-like (hard).

There are several means for specifying beaten pulp. One of those means consists of evaluating the weighted average fiber length distribution (weight distribution) between the body portion and microfiber portion of beaten pulp. Since length distribution of the microfiber portion appears where it is shorter than the distribution of fiber length of the main body portion, by investigating the distribution of fiber length throughout the entire beaten pulp, it is possible to determine the weighted average fiber length distribution of the main body portion and microfiber portion. In addition, an example of another means for specifying beaten pulp consists of evaluating the beating degree of beaten pulp. The value of beating degree tends to become smaller as fibrillation progresses, namely as beating progresses.

Furthermore, weighted average fiber length distribution can be measured as described in Japanese Unexamined Patent Publication No. 2001-288658. In addition, weighted average fiber length distribution can be measured using a Kajaani fiber laboratory fiber length measuring instrument manufactured by Metso Automation Inc.

Beating degree has the same meaning as "Canada standard freeness", and can be measured in accordance with "Section 3(1) Canada Standard Freeness Test Method" of the pulp freeness test methods in JIS P 8121:1995.

The general procedure of "Section 3(1) Canada Standard Freeness Test Method" is as indicated below.

(1) Attach a filtration cartridge to the arm of a support stand, close the bottom cover, and open the air cock of the top cover.
(2) Place a graduated cylinder at a location that is able to receive runoff from the bypass.

(3) Accurately weigh out 1000 mL of sample in a 1000 mL graduated cylinder.
(4) Gently pour the contents of the graduated cylinder into the filtration cartridge.
(5) After having poured in the sample, wait 5 seconds and then open the air cock to allow the sample to flow down.
(6) Once runoff from the bypass has stopped, read the amount of runoff from the bypass.
(7) Correct this amount of runoff to a standard concentration of 0.30% and standard temperature of 20°C using the correction tables (Appended Tables 1 and 2), and use the resulting value as the value of Canada standard freeness, or beating degree.

Beaten pulp is fibrillated, has a main body portion and a microfiber portion extending from the main body portion, and has an overall weighted average fiber length preferably within the range 1.0 to 5.0 mm, more preferably within the range of 1.5 mm to 4.0 mm and even more preferably within the range of 2.0 mm to 3.5 mm. If the weighted average fiber length is below 1.0 mm, fiber length becomes short and the nonwoven fabric tends to become paper-like (hard), while if the weighted average fiber length exceeds 5.0 mm, the pulp fibers prior to fibrillation are excessively long, which may cause each of the pulp fibers to become entangled during fibrillation.

Furthermore, "weighted average fiber length" is a value represented by L(w) that can be measured using the aforementioned Kajaani fiber laboratory fiber length measuring instrument.

In the present description, the microfiber portion as related to beaten pulp refers to fibers having a length of 1 mm or less, and the proportion of the microfiber portion based on the weight of the beaten pulp is preferably within the range of 17% by weight to 65% by weight, more preferably within the range of 20% by weight to 60% by weight, and even more preferably within the range of 23% by weight to 55% by weight. If the proportion of the microfiber portion is below the aforementioned ranges, entanglement of fibers in the nonwoven fabric for absorbents of the present invention, and particularly entanglement between the microfiber portion of beaten pulp and other microfiber portions or other fibers, decreases and strength of the nonwoven fabric, and particularly wet strength, tends to decrease, while if pulp is beaten so that the proportion of the microfiber portion exceeds the aforementioned ranges, the length of the main body portion shortens and wet strength of the nonwoven fabric also tends to decrease.

Furthermore, in the present description, the proportion of the microfiber portion refers to the ratio (% by weight) of fibers within the range of 0.00 mm to 1.00 mm in the "distribution of central fiber length" Fr(w) of the "weighted average fiber length distribution" obtained with the aforementioned Kajaani fiber laboratory fiber length measuring instrument.

Beaten pulp preferably has a beating degree of 400 mL to 650 mL and more preferably has a beating degree of 400 mL to 600 mL. Although wet strength of a nonwoven fabric can be increased by allowing beating to proceed (reducing the value of beating degree), if beating is allowed to proceed, the nonwoven fabric becomes paper-like and texture tends to decrease. If the beating degree is excessively high, entanglement between fibers becomes inadequate and strength, and particularly wet strength, tends to decrease.

The weighted average fiber length distribution and beating degree of beaten pulp can be adjusted according to the type of equipment used for beating, the duration of treatment and so forth.

Examples of beaten pulp raw materials include wood pulp such as coniferous tree pulp or deciduous tree pulp, manila hemp, Linder pulp, bamboo pulp and kenaf.

There are no particular limitations on the regenerated cellulose, and examples thereof include viscose rayon and cuprammonium rayon. The nonwoven fabric for absorbents of the present invention can contain one type or two or more types of regenerated cellulose.

The average fiber length of the regenerated cellulose is preferably 3 mm to 13 mm and more preferably 5 mm to 11 mm. If the average fiber length is excessively long, it becomes difficult to maintain the texture of the nonwoven fabric, while if the average fiber length is excessively short, entanglement between fibers is inadequate, and strength of the nonwoven fabric tends to decrease.

The fineness of the regenerated cellulose is preferably 0.6 dtex to 1.7 dtex and more preferably 0.8 dtex to 1.4 dtex. If the fineness is excessively small, the production cost of the regenerated cellulose increases and spinning quality tends to become unstable, while if fineness is excessively large, it become difficult for fiber entanglement to occur and strength tends to decrease.

The nonwoven fabric of the present invention preferably contains beaten pulp and regenerated cellulose at a ratio of 20 parts by weight to 40 parts by weight and 10 parts by weight to 70 parts by weight, respectively, and more preferably at a ratio of 25 parts by weight to 35 parts by weight and 15 parts by weight to 65 parts by weight, respectively.

Since the beaten pulp extends from the surface of the main body portion of the pulp fibers to the microfiber portion, the degree of hydrogen bonding with other fibers is large, thereby enabling high dry strength to be imparted to the nonwoven fabric of the present invention. In addition, since the microfiber portion, which extends from the surface of the main body portion of the pulp fibers, easily becomes entangled with other microfiber portions or other fibers such as regenerated cellulose fibers, it is able to impart high wet strength to the nonwoven fabric of the present invention.

In addition, in the case of nonwoven fabric composed only of regenerated cellulose, when a liquid is absorbed, although the regenerated cellulose fibers swell causing the gaps between fibers to become smaller and tending to result in inferior liquid permeability, by combining the regenerated cellulose with beaten pulp having surface irregularities as a result of undergoing fibrillation, even in the case of having absorbed liquid, gaps between fibers can be secured, thereby making it possible to form a nonwoven fabric having superior liquid permeability.

Furthermore, although both dry strength and wet strength tend to increase as the proportion of beaten pulp increases, since gaps in the nonwoven fabric are filled in by the microfiber portion causing the gaps to decrease in number, liquid permeability tends to be inferior. In addition, if the proportion of beaten pulp is excessively low, strength tends to decrease since the degree of hydrogen bonding and degree of entanglement of the nonwoven fabric decrease.

In another embodiment of the nonwoven fabric for absorbents of the present invention, the nonwoven fabric for absorbents further contains unbeaten pulp. Examples of unbeaten pulp include pulp that has not been subjected to free beating or wet beating, or in other words, unbeaten pulp.

Examples of unbeaten pulp include wood pulp such as coniferous tree pulp or deciduous tree pulp, manila hemp, Linder pulp, bamboo pulp and kenaf. An example of coniferous tree pulp is coniferous tree bleached kraft pulp. The unbeaten pulp may be of the same material or different material from the material of the beaten pulp.

The unbeaten pulp preferably has a beating degree of 700 mL to 800 mL. The unbeaten pulp is able to impart high bulkiness and flexibility to the nonwoven fabric of the present invention.

In the aforementioned embodiment, the nonwoven fabric of the present invention preferably contains unbeaten pulp in an amount of 5% by weight to 70% by weight, and more preferably an amount of 10% by weight to 60% by weight, based on the weight of the nonwoven fabric.

As a result of the nonwoven fabric containing unbeaten pulp within the aforementioned ranges, the nonwoven fabric is able to have high dry strength and liquid diffusivity. Although the unbeaten pulp is able to impart dry strength to the nonwoven fabric, since it has hardly any microfiber portion, the degree of entanglement is low and is therefore unable to have hardly any effect on wet strength.

In an embodiment in which the nonwoven fabric for absorbents contains unbeaten pulp, the nonwoven fabric preferably contains 20% by weight to 40% by weight of beaten pulp, 10% by weight to 70% by weight of regenerated cellulose and 5% by weight to 70% by weight of unbeaten pulp, and more preferably contains 20% by weight to 40% by weight of beaten pulp, 15% by weight to 65% by weight of regenerated cellulose and 10% by weight to 60% by weight of unbeaten pulp from the viewpoints of strength, liquid permeability and liquid diffusivity.

The nonwoven fabric of the present invention may also contain synthetic fibers within a range that does not impair the diffusing properties of the present invention.

The nonwoven fabric of the present invention preferably has a basis weight of 15 g/m$^2$ to 100 g/m$^2$, more preferably 20 g/m$^2$ to 90 g/m$^2$ and even more preferably 30 g/m$^2$ to 80 g/m$^2$. If the basis weight is less than 15 g/m$^2$, there is the risk of the occurrence of tearing while wearing. In addition, if the basis weight exceeds 100 g/m$^2$, liquid permeability tends to decrease.

The liquid permeation time of the nonwoven fabric of the present invention as determined in a liquid permeability test is preferably 110 seconds or less, more preferably 100 seconds or less and even more preferably 90 seconds or less.

If the liquid permeation time is within the aforementioned ranges, liquid such as body fluid that has reached an absorbent article is able to rapidly permeate in the direction of thickness of the absorbent, namely permeate inside, thereby making it possible to rapidly trap body fluid in the absorbent in combination with the degree of liquid diffusivity.

A liquid permeability test can be carried out in the manner described below.

(1) A liquid impermeable bottom sheet measuring 106 mm × 106 mm × 3 mm (length × width × thickness) is prepared and holes having a diameter of 1 mm are provided at four locations at the corners of a square measuring 11 mm on a side and at one location in the center of the square. Furthermore, holes are provided so that the center of the bottom sheet aligns with the center of the square. In addition, the bottom sheet is preliminarily moistened with artificial urine.

(2) A sample measuring 100 mm × 100 mm is prepared and placed on the bottom sheet so that the center of the sample aligns with the center of the bottom sheet.

(3) A silicone rubber packing having an outer diameter of 50 mm, an inner diameter of 40 mm and a thickness of 3 mm is prepared, and the silicone rubber packing is placed on the sample so that the center of the bottom sheet aligns with the center of the silicone rubber packing.

(4) A weight having a cylindrical portion (inner diameter: 30 mm, total weight: 2 kg) for dropping the artificial urine is placed on the silicone rubber packing so that the center of the lower sheet lies on the central axis line of the cylindrical portion.

(5) 100 mL of artificial urine is dropped onto the sample from the cylindrical portion and the amount of time from immediately after starting of dropping until artificial urine is no longer present on the upper surface of the sample is measured. More precisely, the time at which artificial urine is no longer present within a range having a radius of 14 mm centered on the point that intersects with the central axis line of the cylindrical portion on the upper surface of the sample is determined to be the point at which artificial urine is no longer present.

(6) The aforementioned experiment is repeated five times and the average value thereof is taken to be the liquid permeation time.

The aforementioned experiment is carried out at 20°C.

Artificial urine can be prepared by dissolving 200 g of urea, 80 g of sodium chloride, 8 g of magnesium sulfate, 3 g of calcium chloride and about 1 g of pigment in the form of Blue No. 1 in 10 liters of ion exchange water.

Although the nonwoven fabric of the present invention demonstrates superior strength, liquid permeability and liquid diffusivity even without containing a binder, in another embodiment of the nonwoven fabric of the present invention, the nonwoven fabric may contain a binder in order to further enhance wet strength and the like.

Examples of binders include alkyl celluloses such as carboxymethyl cellulose, methyl cellulose, ethyl cellulose or benzyl cellulose, polyvinyl alcohol, modified polyvinyl alcohol containing a sulfonate group or carboxyl group, and polyamide-epichlorohydrin. The binder can be coated onto a fiber web or nonwoven fabric using a silk screen coater and the like. In the case the binder is water-swellable or non-water-soluble, the binder can be mixed in during production of the fiber web of the nonwoven fabric.

According to the present invention, in a wet spunlace nonwoven fabric composed of cellulosic fibers, in a state of having regular ridges and grooves and the site A, the site B and the site C being in mutual contact, the proportions of fibers occupying the cross-sectional structure of the ridges is such that the proportion at site A is greater than that at site B and the proportion at site B is greater than that at site C, or a site D is present that is in contact with adjacent sites B, the proportion of fibers occupying the cross-sectional area of site D is greater than that of site C, and the liquid absorption height of artificial urine after 5 minutes in terms of Klemm's water absorbency is 130 mm or more, the water absorption multiple is 2.4 times or more and the density of 50 mg/cm$^2$ to 200 mg/cm$^2$, and as a result of making dry strength in the lengthwise direction to be 7 N/25 mm or more and wet strength to be 0.4 N/25 mm or more, effects are obtained of being able to demonstrate high suctioning height and transport a large amount of water while also demonstrating flexibility and being able to withstand use in an absorbent.

Furthermore, the nonwoven fabric of the present invention may have a large number of micropores in the grooves.

The nonwoven fabric for absorbents of the present invention is used as an absorbent of an absorbent article such as a disposable diaper. Preferably, the nonwoven fabric of the present invention is used as an absorbent in combination with a highly water-absorbent polymer.

The absorbent article of the present invention is an absorbent article containing a liquid permeable top sheet, a liquid impermeable back sheet and an absorbent interposed between the top sheet and the back sheet, and is characterized in that the absorbent contains the aforementioned nonwoven fabric for absorbents. Preferably, the absorbent contains the aforementioned nonwoven fabric for absorbents and a highly water-absorbent polymer. The absorbent may further contain pulp.

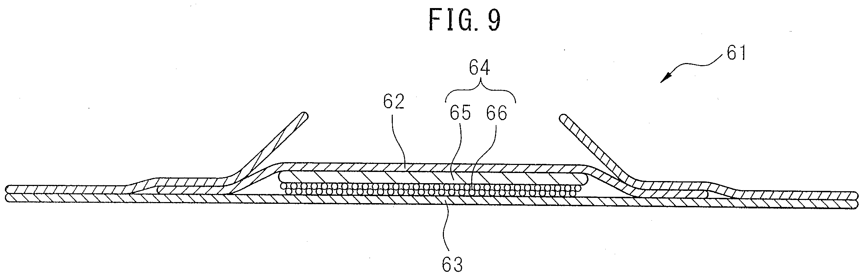

Although a transverse cross-sectional view of an example of the absorbent article of the present invention is shown in FIG. 9, the absorbent article is not limited to that shown in this drawing. In this example, an absorbent article 61 is composed of a liquid permeable top sheet 62, a liquid impermeable back sheet 63 and an absorbent 64, and the absorbent 64 is composed of nonwoven fabric for absorbents 65 and a highly water-absorbent polymer 66. In this example, although only a single nonwoven fabric for absorbents is used, a plurality of nonwoven fabrics for absorbents may also be used. In addition, a configuration may also be employed in which the highly water-absorbent polymer is interposed between two nonwoven fabrics for absorbents. In addition, the highly water-absorbent polymer may be wrapped in the nonwoven fabric for absorbents. The nonwoven fabric for absorbents and the highly water-absorbent polymer may also be wrapped with another sheet. Examples

Comparative Example 1

A mixture of 45% by weight of pulp (manufacturer: Harmac Pacific Inc., weighted average fiber length L(w) as measured with a Kajaani fiber laboratory fiber length measuring instrument: 2.81 mm, proportion of microfiber portion: 11.6% by weight, beating degree: approx. 730 mL), 30% by weight of beaten pulp (obtained by fibrillating the aforementioned pulp using a disc refiner, weighted average fiber length L(w) as measured with a Kajaani fiber laboratory fiber length measuring instrument: 2.79 mm, proportion of microfiber portion: 25.8% by weight, beating degree: 600 mL) and 25% by weight of rayon (fineness: 1.1 dtex, fiber length: 8 mm) was dispersed in a water bath measuring 400 mm × 400 mm (width × depth) followed by manually forming into a sheet to form a fiber web having a basis weight of 50 g/m$^2$ on an 80 mesh wire mesh sheet.

The fiber web still containing water added during sheet formation was subjected to water jet treatment (conveying speed: 30 m/min, water jet pressure: 2 MPa × 1 time + 6 MPa × 3 times, nozzle aperture: 92 μm, nozzle pitch: 0.5 mm, single row).

Next, the fiber web was dried by passing through a hot air conveyor dryer (conveying speed: 1.7 m/min, temperature: 125°C) twice to obtain a single layer structure nonwoven fabric.

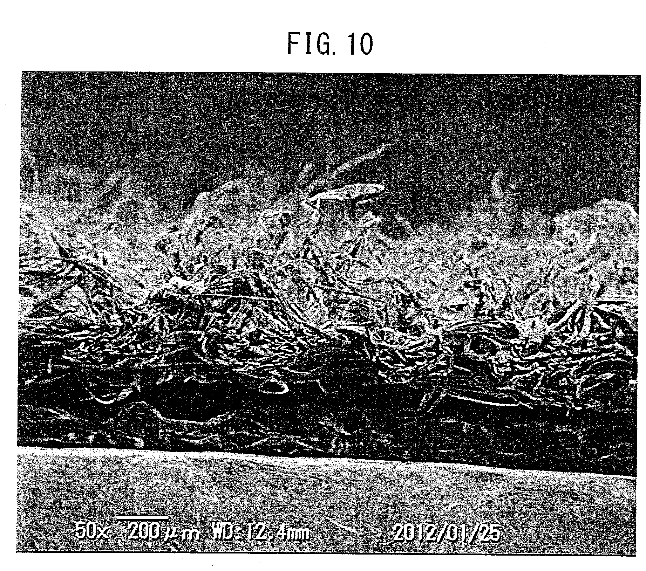

A photograph of a cross-section in the widthwise direction of the resulting nonwoven fabric is shown in FIG. 10.

Comparative Example 2

A mixture of 45% by weight of pulp (manufacturer: Harmac Pacific Inc., weighted average fiber length L(w) as measured with a Kajaani fiber laboratory fiber length measuring instrument: 2.81 mm, proportion of microfiber portion: 11.6% by weight, beating degree: approx. 730 mL), 30% by weight of beaten pulp (obtained by fibrillating the aforementioned pulp using a disc refiner, weighted average fiber length L(w) as measured with a Kajaani fiber laboratory fiber length measuring instrument: 2.79 mm, proportion of microfiber portion: 25.8% by weight, beating degree: 600 mL) and 25% by weight of rayon (fineness: 1.1 dtex, fiber length: 8 mm) was dispersed in a water bath measuring 400 mm $\times$ 400 mm (width $\times$ depth) followed by manually forming into a sheet to form a fiber web having a basis weight of 50 g/m$^2$ on an 80 mesh wire mesh sheet.

The fiber web still containing water added during sheet formation was subjected to water jet treatment (conveying speed: 30 m/min, water jet pressure: 2 MPa $\times$ 1 time, nozzle aperture: 92 $\mu$m, nozzle pitch: 0.5 mm, single row).

Next, the fiber web that underwent water jet treatment was dried with a rotary dryer (conveying speed: 1.5 m/min, temperature: 130°C, blanket contact force: strong) or a hot air dryer (conveying speed: 1.7 m/min, temperature: 125°C, passed through twice).

The fiber web dried with the rotary dryer and the fiber web dried with the hot air conveyor dryer were moistened with a small amount of water followed by superimposing both fiber webs and carrying out water jet treatment on the side having the fiber web dried with the rotary dryer (conveying speed: 30 m/min, water jet pressure: 2 MPa $\times$ 1 time, nozzle aperture: 92 $\mu$m, nozzle pitch: 0.5 mm, single row).

Following water jet treatment, the fiber web was dried with a hot air conveyor dryer (conveying speed: 1.7 m/min, temperature: 125°C, passed through twice) to obtain a bilayer structure nonwoven fabric.

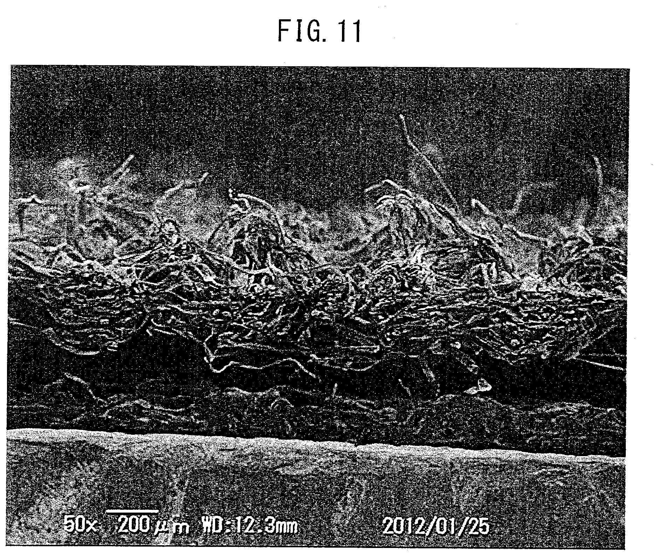

A photograph of a cross-section in the widthwise direction of the resulting nonwoven fabric is shown in FIG. 11.

Example 1

A non-woven fabric was obtained by spraying water onto the nonwoven fabric of Comparative Example 1 (water pressure: 0.6 MPa) followed by subjecting to steam jet treatment (nozzle: 0.5 mm diameter, 2 mm pitch, two rows, conveying speed: 70 m/min, pressure: 0.4 MPa) and then drying by passing once through a hot air conveyor dryer (conveying speed: 1.7 m/min, temperature: 125°C) to obtain a nonwoven fabric.

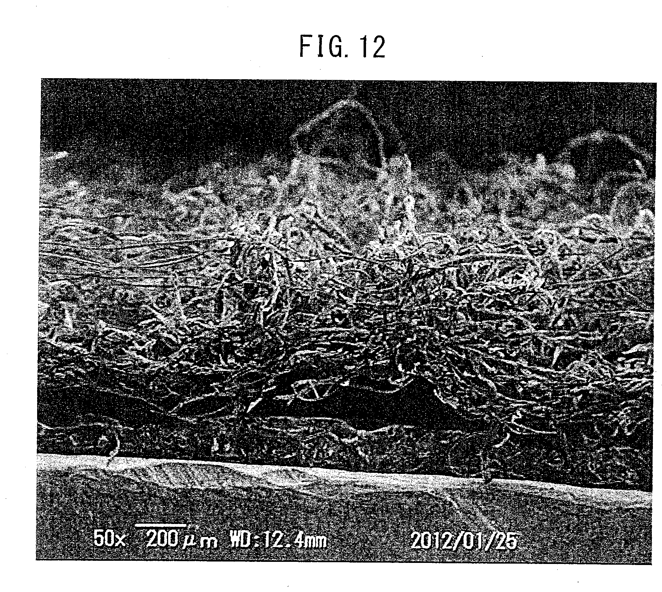

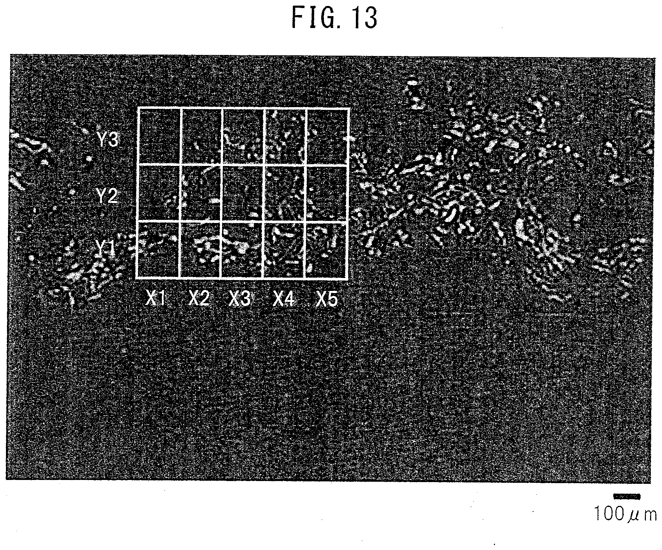

A photograph of a cross-section in the widthwise direction of the resulting nonwoven fabric is shown in FIG. 12 while an XY topographical image thereof is shown in FIG. 13.

Based on the XY topographical image divided into 15 sections, sections X1Y1, X2Y1, X3Y1, X4Y1 and X5Y1 corresponded to site A, sections X1Y2, X1Y3, X2Y3, X3Y3, X4Y3, X5Y3 and X5Y2 corresponded to site B, sections X2Y2, X3Y2 and X4Y2 corresponded to site C, the proportion of fibers occupying the cross-sectional area of site A was 11.2%, the proportion of fibers occupying the cross-sectional area of site B was 9.5%, the proportion of fibers occupying the cross-sectional area of site C was 5.0%, and the average proportion of fibers occupying the cross-sectional area of all cross-sections was 8.7%.

Incidentally, FIG. 5 is an image obtained during the course of calculating the average proportion of fibers occupying the cross-sectional area of the nonwoven fabric of Example 1, with FIG. 5(a) indicating a three-dimensional image, FIG. 5(b) indicating a three-dimensional image observed from overhead on an angle, FIG. 5(c) indicating an XY cross-section of the three-dimensional image, and FIG. 5(d) indicating an XY topographical image.

Example 2

A non-woven fabric was obtained by spraying water onto the nonwoven fabric of Comparative Example 1 (water pressure: 0.6 MPa) followed by subjecting to steam jet treatment (nozzle: 0.5 mm diameter, 2 mm pitch, two rows, conveying speed: 70 m/min, pressure: 0.6 MPa) and then drying by passing once through a hot air conveyor dryer (conveying speed: 1.7 m/min, temperature: 125°C) to obtain a nonwoven fabric.

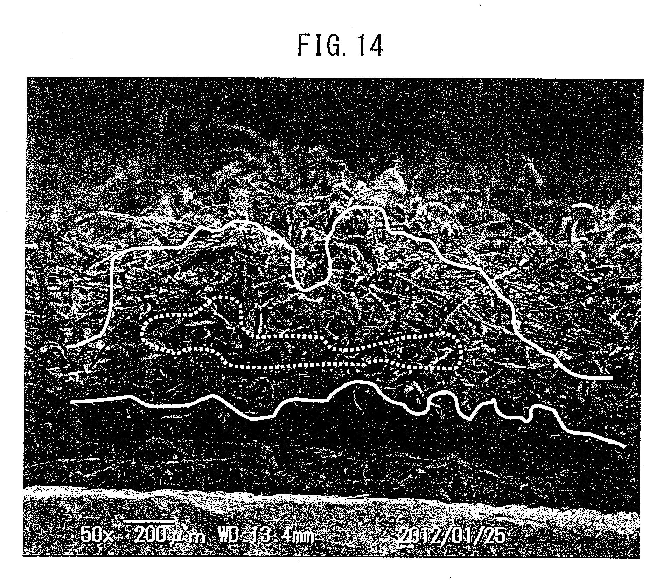

A photograph of a cross-section in the widthwise direction of the resulting nonwoven fabric is shown in FIG. 14 while an XY topographical image thereof is shown in FIG. 15.

Based on the XY topographical image divided into 15 sections, sections X1Y1, X2Y1, X3Y1, X4Y1 and X5Y1 corresponded to site A, sections X1Y2, X1Y3, X2Y3, X3Y3, X4Y3, X5Y3 and X5Y2 corresponded to site B, sections X2Y2, X3Y2 and X4Y2 corresponded to site C, the proportion of fibers occupying the cross-sectional area of site A was 13.4%, the proportion of fibers occupying the cross-sectional area of site B was 7.8%, the proportion of fibers occupying the cross-sectional area of site C was 6.1%, and the average proportion of fibers occupying the cross-sectional area of all cross-sections was 9.1%.

Example 3

After preparing a uniformly dispersed fiber suspension containing the pulp, beaten pulp and rayon used in Comparative Example 1 at a weight ratio of 45:30:25, the suspension was dehydrated with a tanmo paper machine.
The formed fiber web was transported by a conveyor and subjected to water jet treatment on the conveyor (conveying speed: 70 m/min, water jet pressure: 6 MPa $\times$ 2 times, nozzle aperture: 92 $\mu$m, nozzle pitch: 0.5 mm, two rows).
Following water jet treatment, the fiber web was dried with a Yankee dryer (conveying speed: 70 m/min, temperature: 130°C). Furthermore, the fiber web that underwent water jet treatment was passed between the Yankee dryer and a blanket.
After spraying water onto the fiber web that had been dried with the Yankee dryer (water pressure: 0.6 MPa), steam jet treatment was carried out (nozzles: 0.5 mm diameter, 2 mm pitch, two rows, conveying speed: 70 m/min, steam jet pressure: 0.43 MPa, steam jet temperature: 155°C, steam nozzle temperature: 180°C, suction pressure: 4.1 kPa) followed by drying by passing once through a hot air conveyor dryer (conveying speed: 1.7 m/min, temperature: 125°C) and winding to obtain a nonwoven fabric.
An XY topographical image of the resulting nonwoven fabric is shown in FIG. 16.
Based on the XY topographical image divided into 15 sections, sections X1Y1, X2Y1, X3Y1, X4Y1 and X5Y1 corresponded to site A, sections X1Y2, X1Y3, X2Y3, X4Y3, X5Y3 and X5Y2 corresponded to site B, sections X3Y2 and X3Y3 corresponded to site D, sections X2Y2 and X4Y2 corresponded to site C, the proportion of fibers occupying the cross-sectional area of site A was 12.6%, the proportion of fibers occupying the cross-sectional area of site B was 9.4%, the proportion of fibers occupying the cross-sectional area of site D was 4.6%, the proportion of fibers occupying the cross-sectional area of site C was 3.4%, and the average proportion of fibers occupying the cross-sectional area of all cross-sections was 8.6%.

Example 4

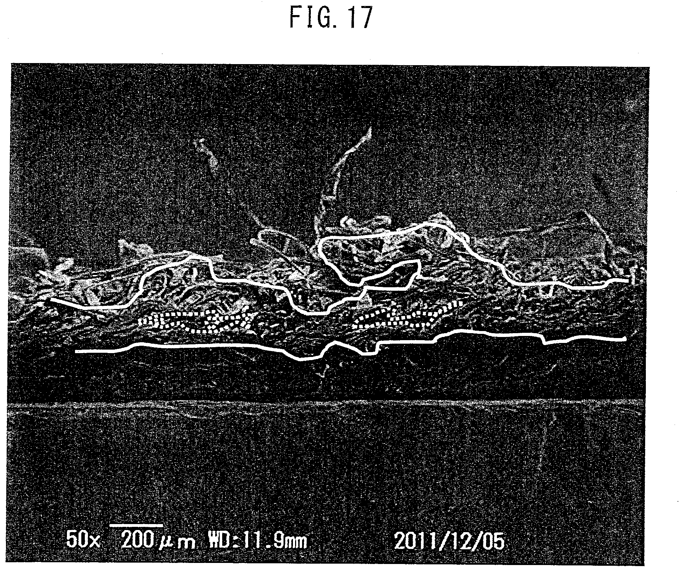

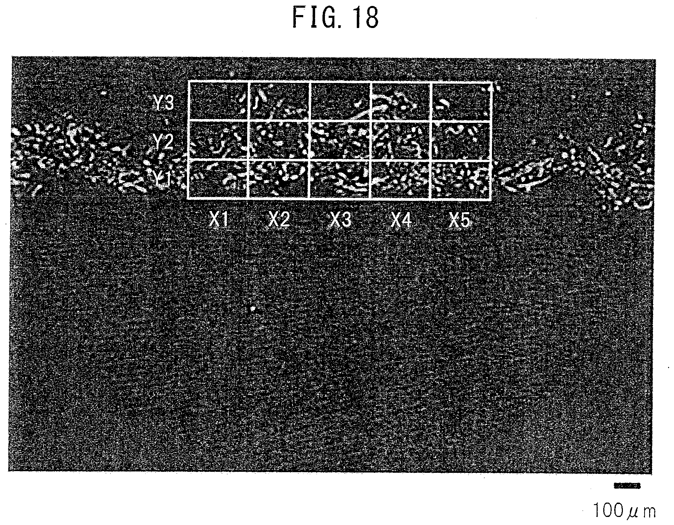

A nonwoven fabric was obtained in the same manner as Example 3 with the exception of changing the conditions of steam jet treatment to a steam jet pressure of 0.6 MPa, steam jet temperature of 159°C, steam nozzle temperature of 186°C and suction pressure of 3.4 kPa, and winding while pressing a nip roll against the fiber web during winding.
A photograph of a cross-section in the widthwise direction of the resulting nonwoven fabric is shown in FIG. 17 while an XY topographical image thereof is shown in FIG. 18.
Based on the XY topographical image divided into 15 sections, sections X1Y1, X2Y1, X3Y1, X4Y1 and X5Y1 corresponded to site A, sections X1Y2, X1Y3, X2Y3, X3Y3, X4Y3, X5Y3 and X5Y2 corresponded to site B, sections X2Y2, X3Y2 and X4Y2 corresponded to site C, the proportion of fibers occupying the cross-sectional area of site A was 15.4%, the proportion of fibers occupying the cross-sectional area of site B was 12.2%, the proportion of fibers occupying the cross-sectional area of site C was 12.0%, and the average proportion of fibers occupying the cross-sectional area of all cross-sections was 13.4%.
Although this nonwoven fabric collapsed due to the nip roll pressure applied during winding after drying, as shown in the following Table 1, it demonstrated a high liquid absorption height and water absorption multiple despite the occurrence of collapse.
Basis weight, thickness, density, liquid absorption height, water absorption multiple, dry strength, dry elongation, wet strength and wet elongation were measured for the nonwoven fabrics obtained in Comparative Examples 1 and 2 and in Examples 1 to 4. The measurement results are shown in Table 1. The methods used to measure each of the measured parameters are as subsequently described.

Table 1

| | Comp.Ex.1 | Comp.Ex.2 | Ex.1 | Ex.2 | Ex.3 | Ex.4 |
|---|---|---|---|---|---|---|
| Basis weight (g/m$^2$) | 56.8 | 60.4 | 60.9 | 54.0 | 53.5 | 55.5 |
| Thickness (mm) | 0.82 | 0.80 | 0.88 | 0.99 | 0.77 | 0.48 |
| Density (mg/cm$^3$) | 70 | 75 | 69 | 54 | 69 | 116 |
| Liquid absorption height (mm) | 146 | 160 | 174 | 169 | 189 | 177 |
| Water absorption multiple (times) | 3.29 | 3.80 | 4.88 | 5.27 | 4.54 | 4.18 |
| Dry strength (N/25 mm) | 9.0 | 12.4 | 8.8 | 5.1 | 13.0 | 10.8 |

(continued)

|  | Comp.Ex.1 | Comp.Ex.2 | Ex.1 | Ex.2 | Ex.3 | Ex.4 |
|---|---|---|---|---|---|---|
| Dry elongation (%) | 9.3 | 18.0 | 14.7 | 12.3 | 3.9 | 4.4 |
| Wet strength (N/25 mm) | 1.9 | 3.1 | 1.3 | 0.8 | 0.8 | 1.4 |
| Wet elongation (%) | 35.0 | 30.0 | 25.3 | 16.7 | 6.7 | 16.5 |

[Basis Weight]

Ten samples measuring 1100 mm $\times$ 100 mm were collected followed by weighing each sample and calculating the basis weight (g/m$^2$) of each sample by dividing the weight (g) of each sample by the area (m$^2$) of each sample. The average value of basis weight of a total of 10 samples was calculated and the resulting average value was used for the value of basis weight.

[Thickness]

The thickness of the nonwoven fabrics was measured using the UF-60 Thickness Gauge manufactured by Daiei Kagaku Seiki Mfg. Co., Ltd. Thickness was measured with the UF-60 using a measuring surface diameter of 44 mm while applying pressure of 0.3 kPa to the nonwoven fabric.

[Density]

Density of the nonwoven fabrics was calculated by dividing the basis weight of the nonwoven fabric by the thickness thereof.

[Water Absorbency Test according to Klemm Method (Liquid Absorption Height, Water Absorption Multiple)]

(1) A sample was cut to a size of 230 mm $\times$ 25 mm (length $\times$ width) followed by measuring the initial weight ($W_0$) thereof.
(2) Artificial urine was filled to a height of 35 mm in a box-shaped container measuring 170 mm $\times$ 90 mm $\times$ 40 mm (length $\times$ width $\times$ depth).
(3) The sample was attached to a suspending apparatus and the end in the lengthwise direction was immersed in the artificial urine to a depth of 30 mm followed by allowing to stand for 5 minutes.
(4) After 5 minutes, the height to which the artificial urine rose was measured and that height was taken to be the "liquid absorption height".
(5) Next, the sample was removed from the suspending apparatus and the portion having a length of 30 mm that had been immersed in the artificial urine was cut off followed by measurement of sample weight ($W_1$).
(6) The water absorption multiple (X) was calculated in accordance with the equation indicated below.

$$X = \{(W_1 \times 230/200) - W_0\}/W_0$$

(7) The aforementioned experiment was repeated five times and the average value thereof was used for the value of water absorption multiple.

Furthermore, the artificial urine was prepared by dissolving 200 g of urea, 80 g of sodium chloride, 8 g of magnesium sulfate, 3 g of calcium chloride and about 1 g of pigment in the form of Blue No. 1 in 10 liters of ion exchange water.

[Dry Strength]

Dry strength refers to the tensile strength (N) during cutting when a sample measuring 25 mm wide $\times$ 150 mm long cut from a produced non-woven fabric so that the lengthwise direction thereof is the machine direction is dried by allowing to stand for 24 hours under conditions of 20°C and relative humidity of 65% followed by measuring the dried

# EP 2 840 175 B1

sample with a Tensilon tensile tester at a chuck interval of 100 mm and pulling speed of 200 mm/min. Dry strength was measured using the Model 5564 Testing Machine manufactured by Instron Corp., and the average value of five measurements was used for the value of dry strength.

[Dry Elongation]

Dry elongation was measured simultaneous to measurement of dry strength, and refers to elongation (%) of a sample that demonstrates tensile strength (N) during cutting when the sample is cut to a size of 25 mm wide × 150 mm long from a produced nonwoven fabric so that the lengthwise direction is the machine direction and dried by allowing to stand for 24 hours at 20°C and relative humidity of 65% followed by measuring the dried sample with a Tensilon tensile tester at a chuck interval of 100 mm and pulling speed of 200 mm/min, and is expressed as a percentage obtained by dividing the elongation of the sample by the chuck interval of 100 mm.

[Wet Strength]

Wet strength refers to the tensile strength (N) during cutting when a sample, measuring 25 mm wide × 150 mm long cut from a produced non-woven fabric so that the lengthwise direction thereof is the machine direction and immersed in distilled water for 5 minutes followed by allowing to stand for 1 minute on a stainless steel, plain woven 23 mesh wire mesh, is measured with a Tensilon tensile tester at a chuck interval of 100 mm and pulling speed of 200 mm/min under conditions of 20°C and relative humidity of 65%. Wet strength was measured using the Model 5564 Testing Machine manufactured by Instron Corp., and the average value of five measurements was used for the value of wet strength.

[Wet Elongation]

Wet elongation was measured simultaneous to measurement of wet strength, and refers to elongation (%) of a sample that demonstrates tensile strength (N) during cutting when the sample, cut to a size of 25 mm wide × 150 mm long from a produced nonwoven fabric so that the lengthwise direction is the machine direction and immersed for 5 minutes in distilled water followed by allowing to stand for 1 minute on a stainless steel, plain woven 23 mesh wire mesh, is measured with a Tensilon tensile tester at a chuck interval of 100 mm and pulling speed of 200 mm/min under conditions of 20°C and relative humidity of 65%, and is expressed as a percentage obtained by dividing the elongation of the sample by the chuck interval of 100 mm.

INDUSTRIAL APPLICABILITY

The nonwoven fabric of the present invention can be used as an absorbent of an absorbent article and the like. The absorbent article of the present invention can be used as a disposable diaper.

BRIEF DESCRIPTION OF THE REFERENCE SYMBOLS

1 Nonwoven fabric
2 Ridge
2c Central portion of ridge in widthwise direction
2f Top of ridge
2w Sidewall of ridge
3,3' Groove
10 Nonwoven fabric production apparatus
11 Raw material supply head
12 Water jet nozzle
13 Suction box
14 Steam nozzle
15 Suction drum
16 Web forming conveyor
17 Suction pickup
18 Web transport conveyor
19 Web transport conveyor

20 Drying drum
30 Web
51 Steam jet
52 Groove
61 Absorbent article
62 Top sheet
63 Back sheet
64 Absorbent
65 Nonwoven fabric for absorbents
66 Highly water-absorbent polymer
A Site A
B Site B
C Site C
D Site D

**Claims**

1. A nonwoven fabric (1) for absorbents composed of hydrophilic fibers in the form of cellulosic fibers, wherein

   the non-woven fabric (1) has an upper side and a lower side on the opposite side therefrom,
   the non-woven fabric (1) has ridges (2) and grooves (3) in the upper surface extending in the lengthwise direction that alternate in the widthwise direction, wherein
   when a rectangle that contacts outermost portions of a ridge (2) is drawn in a topographical image of a cross-section in the widthwise direction of the nonwoven fabric obtained by X-ray CT, and then the rectangle is divided into three sections in the vertical direction, the three sections being referred to as Y1, Y2 and Y3, respectively, from the bottom, and is divided into five sections in the horizontal direction, the five sections being referred to as X1, X2, X3, X4 and X5, respectively, from the left, and sections X1Y1, X2Y1, X3Y1, X4Y1 and X5Y1 are defined as a site A, sections X1Y2, X1Y3, X2Y3, X4Y3, X5Y3 and X5Y2 are defined as a site B, and sections X2Y2 and X4Y2 are defined as a site C, and
   the proportion of fibers occupying the cross-sectional area of the site A is higher than the proportion of fibers occupying the cross-sectional area of the site B, and
   the proportion of fibers occupying the cross-sectional area of the site B is higher than the proportion of fibers occupying the cross-sectional area of the site C, wherein the proportion of fibers occupying the cross-sectional area of the groove (3) is higher than the proportion of fibers occupying the cross-sectional area of the site B.

2. The nonwoven fabric according to claim 1, wherein, when sections X3Y2 and X3Y3 are defined as a site D, the proportion of fibers occupying the cross-sectional area of the site D is smaller than the proportion of fibers occupying the cross-sectional area of the site B and larger than the proportion of fibers occupying the cross-sectional area of the site C.

3. The nonwoven fabric according to claims 1 or 2, wherein tensile strength in the lengthwise direction is 4 N/25 mm or more when dry and 0.4 N/25 mm or more when wet.

4. The nonwoven fabric according to claim 1 or 3, wherein when the average proportion of fibers occupying the cross-sectional area of all cross-sections in the widthwise direction of the nonwoven fabric is defined as $r_0$, then the proportion of fibers occupying the cross-sectional area of the site A ($r_A$) is $1.2r_0$ or more, the proportion of fibers occupying the cross-sectional area of the site B ($r_B$) is $0.8r_0$ to less than $1.2r_0$, and the proportion of fibers occupying the cross-sectional area of the site C ($r_C$) is less than $0.8r_0$.

5. The nonwoven fabric according to claims 2 or 3, wherein when the average proportion of fibers occupying the cross-sectional area of all cross-sections in the widthwise direction of the nonwoven fabric (1) is defined as $r_0$, then the proportion of fibers occupying the cross-sectional area of the site A ($r_A$) is $1.2r_0$ or more, the proportion of fibers occupying the cross-sectional area of the site B ($r_B$) is $0.8r_0$ to less than $1,2r_0$, the proportion of fibers occupying the cross-sectional area of site D ($r_D$) is $0.5r_0$ to less than $0.8r_0$, and the proportion of fibers occupying the cross-sectional area of the site C ($r_C$) is less than $0.5r_0$.

6. The nonwoven fabric according to any of claims 1 to 5, wherein the nonwoven fabric (1) contains beaten pulp and regenerated cellulose at ratios of 20 parts by weight to 40 parts by weight and 10 parts by weight to 70 parts by weight, respectively, and the beaten pulp is fibrillated and has a weighted average fiber length over a range of 1.0 mm to 5.0 mm.

7. An absorbent article containing a liquid permeable top sheet (62), a liquid impermeable back sheet (63) and an absorbent (64) interposed between the top sheet (62) and the back sheet (63), wherein the absorbent (64) contains the nonwoven fabric (1) according to any of claims 1 to 6.

**Patentansprüche**

1. Ein Faservliesstoff (1) für Absorptionsmittel, die aus wasserbindenden Fasern in der Form von zellulosischen Fasern gebildet sind, wobei

der Faservliesstoff (1) eine Oberseite und eine Unterseite auf der davon gegenüberliegenden Seite aufweist, der Faservliesstoff (1) Grate (2) und Nuten (3) in der oberen Oberfläche aufweist, die sich in der Längsrichtung erstrecken, die in der Breitenrichtung alternieren, wobei wenn ein Rechteck, das äußerste Teile eines Grats (2) kontaktiert, in ein topographisches Bild eines Schnitts in der Breitenrichtung des Faservliesstoffs gezeichnet wird, das mittels computertomographischer Röntgenstrahlung erhalten wurde, und dann das Rechteck in drei Abschnitte in der Vertikalrichtung geteilt ist, die drei Abschnitte als Y1, Y2 und Y3 bezeichnet werden, jeweils, von unten, und in fünf Abschnitte in der horizontalen Richtung geteilt ist, die fünf Abschnitte als X1, X2, X3, X4 und X5 bezeichnet werden, jeweils, von links, und Abschnitte X1Y1, X2Y1, X3Y1, X4Y1 und X5Y1 als eine Lage A definiert sind, Abschnitte X1Y2, X1Y3, X2Y3, X4Y3, X5Y3 und X5Y2 als eine Lage B definiert sind, und Abschnitte X2Y2 und X4Y2 als eine Lage C definiert sind, und der Anteil an Fasern, die die Schnittfläche der Lage A besetzen, höher ist als der Anteil an Fasern, die die Schnittfläche der Lage B besetzen, und der Anteil an Fasern, die die Schnittfläche der Lage B besetzen, höher ist als der Anteil an Fasern, die die Schnittfläche der Lage C besetzen, wobei der Anteil an Fasern, die die Schnittfläche der Nut (3) besetzen, höher ist als der Anteil an Fasern, die die Schnittfläche der Lage B besetzen.

2. Der Faservliesstoff gemäß Anspruch 1, wobei, wenn Bereiche X3Y2 und X3Y3 als eine Lage D definiert werden, der Anteil an Fasern, die die Schnittfläche der Lage D besetzen, kleiner ist als der Anteil an Fasern, die die Schnittfläche der Lage B besetzen, und höher ist als der Anteil an Fasern, die die Schnittfläche der Lage C besetzen.

3. Der Faservliesstoff gemäß Ansprüchen 1 oder 2, wobei Zugfestigkeit in der Längsrichtung in trockenem Zustand 4 N/25 mm oder höher beträgt und 0.4 N/25 mm oder höher in nassem Zustand.

4. Der Faservliesstoff gemäß Anspruch 1 oder 3, wobei wenn der durchschnittliche Anteil an Fasern, die die Schnittfläche von allen Schnittflächen in der Breitenrichtung des Faservliesstoffs besetzen als ro definiert ist, dann der Anteil an Fasern, die die Schnittfläche der Lage A($r_A$) besetzen, $1.2r_0$ oder mehr beträgt, der Anteil an Fasern, die die Schnittfläche der Lage B($r_B$) besetzen, $0,8r_0$ bis weniger als $1.2r_0$ beträgt, und der Anteil an Fasern, die die Schnittfläche der Lage C($r_C$) besetzen, weniger als $0,8r_0$ beträgt.

5. Der Faservliesstoff gemäß Ansprüchen 2 oder 3, wobei wenn der durchschnittliche Anteil an Fasern, die die Schnittfläche von allen Schnittflächen in der Breitenrichtung des Faservliesstoffs (1) besetzen als $r_0$ definiert ist, dann der Anteil an Fasern, die die Schnittfläche der Lage A($r_A$) besetzen, $1.2r_0$ oder mehr beträgt, der Anteil an Fasern, die die Schnittfläche der Lage B($r_B$) besetzen, $0,8r_0$ bis weniger als $1.2r_0$ beträgt, der Anteil an Fasern, die die Schnittfläche der Lage D($r_D$) besetzen, $0.5r_0$ bis weniger als $0,8r_0$ beträgt, und der Anteil an Fasern, die die Schnittfläche der Lage C($r_C$) besetzen, weniger als $0.5r_0$ beträgt.

6. Der Faservliesstoff gemäß einem der Ansprüche 1 bis 5, wobei der Faservliesstoff (1) gemahlenen Zellstoff und regenerierte Zellulose bei Anteilen von 20 Gewichtsteilen bis 40 Gewichtsteilen und 10 Gewichtsteilen bis 70 Gewichtsteilen umfasst, jeweils, und der gemahlenen Zellstoff fibrilliert ist und eine gewichtete Durchschnittsfaserlänge über einen Bereich von 1.0 mm bis 5.0 mm aufweist.

7. Ein absorbierender Artikel, der eine flüssigkeitsdurchlässige Oberlage (62), eine flüssigkeitsundurchlässige Unterlage (63) und ein Absorptionsmittel (64), das zwischen der Oberlage (26) und der Unterlage (63) eingeschoben ist, umfasst, wobei das Absorptionsmittel (64) den Faservliesstoff (1) gemäß einem der Ansprüche 1 bis 6 umfasst.

**Revendications**

1. Tissu non-tissé (1) pour absorbants constitué de fibres hydrophiles sous forme de fibres cellulosiques, dans lequel

   le tissu non-tissé (1) a un côté supérieur et un côté inférieur du côté opposé à celui-ci,
   le tissu non-tissé (1) présente des nervures (2) et des rainures (3) dans la surface supérieure s'étendant dans le sens de la longueur alternant dans le sens de la largeur, dans lequel
   lorsqu'un rectangle qui entre en contact avec les parties les plus extérieures d'une nervure (2) est tracé dans une image topographique d'une surface transversale dans le sens de la largeur du tissu non-tissé obtenue par tomodensitométrie, le rectangle est alors divisé en trois sections dans la direction verticale, les trois sections étant désignées par Y1, Y2 et Y3 respectivement, depuis le bas, et est divisé en cinq sections dans la direction horizontale, les cinq sections étant désignées respectivement par X1, X2, X3, X4 et X5, depuis la gauche, et les sections X1Y1, X2Y1, X3Y1, X4Y1 et X5Y1 sont définies comme un site A, les sections X1Y2, X1Y3, X2Y3, X4Y3, X5Y3 et X5Y2 sont définies comme un site B et les sections X2Y2 et X4Y2 sont définies comme un site C, et la proportion de fibres occupant la surface transversale du site A est supérieure à la proportion de fibres occupant la surface transversale du site B, et
   la proportion de fibres occupant la surface transversale du site B est supérieure à la proportion de fibres occupant la surface transversale du site C, dans laquelle la proportion de fibres occupant la surface transversale de la rainure (3) est supérieure à la proportion de fibres occupant la surface transversale du site B.

2. Tissu non-tissé selon la revendication 1, dans lequel, lorsque les sections X3Y2 et X3Y3 sont définies comme un site D, la proportion de fibres occupant la surface transversale du site D est inférieure à la proportion de fibres occupant la surface transversale du site B et supérieure à la proportion de fibres occupant la surface transversale du site C.

3. Tissu non-tissé selon la revendication 1 ou 2, dans lequel la résistance à la traction dans le sens de la longueur est de 4 N/25 mm ou plus lorsqu'il est sec et de 0,4 N/25 mm ou plus lorsqu'il est humide.

4. Tissu non-tissé selon la revendication 1 ou 3, dans lequel lorsque la proportion moyenne de fibres occupant la surface transversale de toutes les sections transversales dans le sens de la largeur du tissu non tissé est définie comme étant $r_0$, la proportion de fibres occupant la surface transversale du site A ($r_A$) est alors de $1,2r_0$ ou plus, la proportion de fibres occupant la surface transversale du site B ($r_B$) est de $0,8r_0$ à moins de $1,2r_0$, et la proportion de fibres occupant la surface transversale du site C ($r_C$) est inférieure à $0,8r_0$.

5. Tissu non-tissé selon la revendication 2 ou 3, dans lequel lorsque la proportion moyenne de fibres occupant la surface transversale de toutes les sections transversales dans le sens de la largeur du tissu non-tissé (1) est définie comme étant $r_0$, la proportion de les fibres occupant la surface transversale du site A ($r_A$) est alors de $1,2r_0$ ou plus, la proportion de fibres occupant la surface transversale du site B ($r_B$) est de $0,8r_0$ à moins de $1,2r_0$, la proportion de fibres occupant la surface transversale du site D ($r_D$) est de $0,5r_0$ à moins de $0,8r_0$, et la proportion de fibres occupant la surface transversale du site C ($r_C$) est inférieure à $0,5r_0$.

6. Tissu non-tissé selon l'une quelconque des revendications 1 à 5, dans lequel le tissu non-tissé (1) contient de la pâte battue et de la cellulose régénérée dans des rapports de 20 parties en poids à 40 parties en poids et de 10 parties en poids à 70 parties en poids, respectivement, et la pâte battue est fibrillée et a une longueur de fibre moyenne pondérée sur une plage de 1,0 mm à 5,0 mm.

7. Article absorbant contenant une feuille supérieure perméable aux liquides (62), une feuille arrière imperméable aux liquides (63) et un absorbant (64) intercalé entre la feuille supérieure (62) et la feuille arrière (63), dans lequel l'absorbant (64) contient le tissu non-tissé (1) selon l'une quelconque des revendications 1 à 6.

# FIG. 1

# FIG. 2

# FIG. 3

# FIG. 4

# FIG. 5

（a）

（b）

（c）

（d）

100μm

FIG. 6

# FIG. 7

# FIG. 8

FIG. 9

# FIG. 10

50x 200μm WD:12.4mm        2012/01/25

FIG. 11

## FIG. 12

## FIG. 13

100μm

## FIG. 14

50x  200μm  WD 13.4mm    2012/01/25

# FIG. 15

100μm

# FIG. 16

FIG. 17

# FIG. 18

100μm

**REFERENCES CITED IN THE DESCRIPTION**

**Patent documents cited in the description**

- JP 3021227 B
- JP 2003235894 A
- JP 2001288658 A